# EUROPEAN PATENT APPLICATION

(11) **EP 4 559 891 A1**
(43) Date of publication of application: **28.05.2025**
(21) Application number: 23842903.9
(22) Date of filing: 13.07.2023
(51) Int. Cl.: C07C 29/80, C07C 29/15, C07C 29/88, C07C 31/20, C08G 18/42, C08G 63/12, C12P 7/18

(54) **METHOD FOR PRODUCING 1,6-HEXANE DIOL COMPOSITION, 1,6-HEXANE DIOL COMPOSITION, AND POLYMER**

(30) Priority: 22.07.2022 JP 2022117125
(71) Applicant: DIC Corporation, Tokyo 174-8520 (JP)
(72) Inventor: SATO Kouji, Takaishi-shi, Osaka 592-0001 (JP); TSUTSUMI Hiroshi, Takaishi-shi, Osaka 592-0001 (JP); KITADA Mitsuru, Takaishi-shi, Osaka 592-0001 (JP); MIYAMOTO Masanori, Sakura-shi, Chiba 285-8668 (JP); SHIGEHIRO Tatsuya, Sakura-shi, Chiba 285-8668 (JP)
(74) Representative: TBK
(86) International application number: PCT/JP2023/025826
(87) International publication number: WO 2024/018980

(57) **Abstract**

[Problem] An object of the present invention is to provide an environmentally friendly method for producing a biomass resource-derived 1,6-hexanediol composition having excellent reactivity, a 1,6-hexanediol composition having excellent reactivity, which is obtained by the production method, and a polymer having excellent reactivity, which is obtained by reacting the 1,6-hexanediol composition.

[Means for Resolution] The production method of the present invention relates to a method for producing a 1,6-hexanediol composition, the method including: a step (1) of producing a 1,6-hexanediol composition from 6-hydroxycaproic acid and/or a derivative thereof obtained from a biomass resource-derived raw material; and a step (2) of purifying the 1,6-hexanediol composition obtained in the step (1), by ion exchange and/or distillation.

## Description

### TECHNICAL FIELD

The present invention relates to a method for producing a 1,6-hexanediol composition, a 1,6-hexanediol composition, and a polymer.

### BACKGROUND ART

A 1,6-hexanediol (1,6-HD) composition is an intermediate product useful for the production of polymers such as polyesters and polyurethanes. 1,6-Hexanediol compositions have been conventionally produced by esterifying a carboxylic acid mixture of adipic acid, 6-hydroxycaproic acid, glutaric acid, and the like that are produced by oxidizing cyclohexane, which is a petrochemical product, followed by hydrogenation and distillation purification.

On the other hand, with the increase in environmental awareness in recent years, biomass resource-derived raw materials are desirable, rather than petroleum-derived raw materials that have an impact on global warming, and with regard to 1,6-hexanediol, attempts have been made to produce biomass resource-derived 1,6-hexanediol compositions from biomass resource-derived raw materials using microorganisms; however, no product has been put on the market. For example, PTL 1 and PTL 2 relate to production methods for 1,6-hexanediol compositions using enzymes, and disclose descriptions on the genetic information and metabolic pathways of the enzymes, and production methods by purification.

### CITATION LIST

### PATENT LITERATURE

PTL 1: JP 2020-114227 A
PTL 2: JP 6680671 B2

### SUMMARY OF INVENTION

### TECHNICAL PROBLEM

However, although PTL 1 and PTL 2 disclose environmentally friendly production methods for biomass resource-derived 1,6-hexanediol compositions, these documents do not disclose impurities included in the 1,6-hexanediol compositions, and nothing is mentioned about the reactivity of polymers such as polyesters and polyurethanes produced using the 1,6-hexanediol. The inventors of the present invention produced a 1,6-hexanediol composition according to the description in JP 6680671 B2, and then performed purification using known techniques; however, when a reaction of a polymer such as a polyester or a polyurethane was performed using the 1,6-hexanediol composition, decrease in reactivity and decrease in physical properties such as tensile strength and tensile elongation, which were not observed in conventional petroleum-derived 1,6-hexanediol compositions, were recognized. The present inventors conducted detailed studies, and as a result, it was found that such a phenomenon is observed when the content of a compound having two or more secondary hydroxyl groups in one molecule, such as glucose, which is a raw material for a biomass resource-derived 1,6-hexanediol composition, 6-hydroxyhexanal, and/or a derivative thereof is high.

It is an object of the present invention to solve the above-described problems and to provide an environmentally friendly production method for a biomass resource-derived 1,6-hexanediol composition having excellent reactivity, a 1,6-hexanediol composition having excellent reactivity obtained by the production method, and a polymer such as a polyester or polyurethane having excellent reactivity, which is obtained by reacting the 1,6-hexanediol composition.

### SOLUTION TO PROBLEM

The present inventors conducted intensive studies, and as a result, they found that reactivity can be improved by adjusting the content of a compound having two or more secondary hydroxyl groups in one molecule, 6-hydroxyhexanal, and/or a derivative thereof in a biomass resource-derived 1,6-hexanediol composition to be equal to or less than a specific amount, thus completing the present invention.

That is, the present invention provides the following inventions.
[1] A method for producing a 1,6-hexanediol composition, the method including:
   a step (1) of producing a 1,6-hexanediol composition from 6-hydroxycaproic acid and/or a derivative thereof obtained from a biomass resource-derived raw material; and
   a step (2) of purifying the 1,6-hexanediol composition obtained in the step (1) by ion exchange and/or distillation,
   wherein the 1,6-hexanediol composition contains either or both of 1,6-hexanediol and a 1,6-hexanediol derivative, (A) a compound having two or more secondary hydroxyl groups in one molecule, and (B) 6-hydroxyhexanal and/or a derivative thereof,
   a content of (A) the compound having two or more secondary hydroxyl groups in one molecule with respect to a total amount of the 1,6-hexanediol composition is 100 ppm by mass or less, and
   a total content of (B) 6-hydroxyhexanal and/or a derivative thereof with respect to the total amount of the 1,6-hexanediol composition is 1500 ppm by mass or less.
[2] The method for producing a 1,6-hexanediol composition according to [1], wherein the step (1) is a step of producing the 1,6-hexanediol composition under conditions at 70°C or lower.
[3] The method for producing a 1,6-hexanediol composition according to [1], wherein the step (1) is a step of producing the 1,6-hexanediol composition under conditions at 50°C or lower.
[4] The method for producing a 1,6-hexanediol composition according to any one of [1] to [3], wherein the step (1) is a step of producing the 1,6-hexanediol composition using a microorganism.
[5] A 1,6-hexanediol composition obtained by the method for producing a 1,6-hexanediol composition according to any one of [1] to [4], the 1,6-hexanediol composition containing:
   either or both of 1,6-hexanediol and a 1,6-hexanediol derivative;
   (A) a compound having two or more secondary hydroxyl groups in one molecule; and
   (B) 6-hydroxyhexanal and/or a derivative thereof,
      wherein a total content of (A) the compound having two or more secondary hydroxyl groups in one molecule with respect to a total amount of the 1,6-hexanediol composition is 100 ppm by mass or less, and
      a total content of (B) 6-hydroxyhexanal and/or a derivative thereof with respect to the total amount of the 1,6-hexanediol composition is 1500 ppm by mass or less.
[6] A polymer produced using the 1,6-hexanediol composition according to [5] as a reaction raw material.
[7] The polymer according to [6], wherein the polymer is a polyester or a polyurethane.

### ADVANTAGEOUS EFFECTS OF INVENTION

The method for producing a 1,6-hexanediol composition of the present invention is an environmentally friendly production method that can reduce carbon dioxide emissions and the like because the 1,6-hexanediol composition is produced from biomass resource-derived raw materials. Furthermore, with regard to the 1,6-hexanediol composition of the present invention, since the total content of (A) a compound having two or more secondary hydroxyl groups in one molecule is 100 ppm by mass or less, and the total content of (B) 6-hydroxyhexanal and/or a derivative thereof is 1500 ppm by mass or less, the 1,6-hexanediol composition has excellent reactivity. Therefore, the method for producing a 1,6-hexanediol composition of the present invention can produce a 1,6-hexanediol composition having excellent reactivity in an environmentally friendly manner.

Furthermore, the polymer (polyester or the like) of the present invention obtained by reacting the 1,6-hexanediol composition of the present invention also has excellent reactivity. Furthermore, a polyurethane of the present invention has satisfactory tensile strength and tensile elongation.

### DESCRIPTION OF EMBODIMENTS

First, a 1,6-hexanediol composition obtained by the method for producing a 1,6-hexanediol composition of the present invention (also described as 1,6-hexanediol composition of the present invention) will be described.

### <1,6-Hexanediol composition>

The 1,6-hexanediol composition (may be simply referred to as "composition") of the present invention is a composition containing either or both of 1,6-hexanediol and a 1,6-hexanediol derivative, (A) a compound having two or more secondary hydroxyl groups in one molecule, and (B) 6-hydroxyhexanal and/or a derivative thereof. Incidentally, in the present specification, a secondary hydroxyl group means a hydroxyl group in which the carbon atom to which the hydroxyl group is bonded is a secondary carbon atom.

In the present specification, (A) a compound having two or more secondary hydroxyl groups in one molecule may be simply referred to as "component (A)", and the same also applies to component (B).

### <<1,6-Hexanediol and 1,6-hexanediol derivative>>

The 1,6-hexanediol included in the composition of the present invention is preferably unmodified 1,6-hexanediol; however, a 1,6-hexanediol derivative may also be used. That is, the composition of the present invention contains at least any one of 1,6-hexanediol and a 1,6-hexanediol derivative.

The 1,6-hexanediol derivative included in the composition of the present invention is a compound in which any one or both of the two hydroxyl groups of the 1,6-hexanediol included in the 1,6-hexanediol composition of the present invention have been modified. Here, as a method for modifying the hydroxyl groups of 1,6-hexanediol, any known method for modifying a hydroxyl group can be used, and examples thereof include an etherification reaction, an esterification reaction, and modification by (meth)acrylic acid.

Examples of the 1,6-hexanediol derivative include epoxy group-containing 1,6-hexanediol derivatives such as 1,6-hexanediol diglycidyl ether and 6-hydroxyhexyl glycidyl ether; (meth)acryloyl group-containing 1,6-hexanediol derivatives such as 1,6-hexanediol di(meth)acrylate, 6-hydroxyhexyl acrylate, and 1,6-hexanediol monoacrylate monomethacrylate; vinyl ether group-containing 1,6-hexanediol derivatives such as 1,6-hexanediol divinyl ether and 1,6-hexanediol monovinyl ether; aliphatic alkyl ether group-containing 1,6-hexanediol derivatives such as 6-propyloxy-1-hexanol, 1,6-dipropoxy-hexane, 1,6-hexanediol methyl ether, and 1,6-dimethoxyhexane; and fatty acid ester group-containing 1,6-hexanediol derivatives such as propyl 6-hydroxyhexanoate, di-n-propyl adipate, methyl 6-hydroxycaproate, and dimethyl adipate. These may be used singly, or two or more kinds thereof may be used in combination.

In the present specification, the term (meth)acryloyl group means either or both of an acryloyl group and a methacryloyl group, and the term (meth)acrylate means either or both of acrylate and methacrylate.

The total content of either or both of 1,6-hexanediol and the 1,6-hexanediol derivative in the composition of the present invention is preferably 96.00% to 99.99% by mass, more preferably 98.00% to 99.99% by mass, and even more preferably 99.50% to 99.99% by mass, with respect to the total amount of the composition. By setting the purity of the 1,6-hexanediol composition to be within the above-described range while setting the total content of the component (A) and the total content of the component (B) to be within the above-described ranges, the effects of the present invention tend to be more favorably obtained.

In the present specification, the total content of either or both of 1,6-hexanediol and the 1,6-hexanediol derivative is a value measured by gas chromatography mass spectrometry (GC/MS).

### <<Component (A)>>

The component (A) is not particularly limited as long as it is a compound having two or more secondary hydroxyl groups in one molecule, and examples thereof include aliphatic polyols represented by 2,3-butylene glycol, erythritol, threitol, arabitol, xylitol, ribitol, iditol, galactitol, sorbitol, mannitol, and volemitol; alicyclic polyols represented by 1,2-cyclohexanediol, 1,4-cyclohexanediol, glucose, fructose, inositol, and quercitol; and oligomers obtained by dehydration condensation of any combination of these compounds. These may be used singly or in combination of two or more kinds thereof. Among them, from the viewpoint of having significant influence on the reactivity of the 1,6-hexanediol composition and the reactivity of a polymer such as a polyester obtained by reacting the 1,6-hexanediol composition, the component (A) may be 1,4-cyclohexanediol or glucose.

The total content of (A) the compound having two or more secondary hydroxyl groups in one molecule (preferably, the total content of either or both of 1,4-cyclohexanediol and glucose) with respect to the total amount of the 1,6-hexanediol composition of the present invention is 100 ppm by mass or less, preferably 50 ppm by mass or less, more preferably 10 ppm by mass or less, even more preferably 5 ppm by mass or less, and particularly preferably 0 ppm by mass (not included). This tends to result in satisfactory reactivity of the composition of the present invention, satisfactory reactivity of the polymer (polyester or the like) produced using the composition of the present invention as a reaction raw material, and satisfactory film physical properties (tensile strength and tensile elongation) of polyurethane.

In the present specification, the total content of (A) the compound having two or more secondary hydroxyl groups in one molecule is a value measured by gas chromatography mass spectrometry (GC/MS).

### <<Component (B)>>

The component (B) is not particularly limited as long as it is either or both of 6-hydroxyhexanal and a derivative of 6-hydroxyhexanal.

The derivative of 6-hydroxyhexanal is not particularly limited as long as it is a derivative of 6-hydroxyhexanal (compound represented by the following Formula (1)), and examples thereof include a cyclization product, an aldol condensation product, a glycerin reaction product, and a hexanediol (HDO) reaction product, while specific examples thereof include compounds represented by the following Formula (2) to Formula (7). These may be used singly, or two or more kinds thereof may be used in combination.
[Chemical Formula 1]

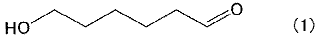

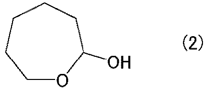

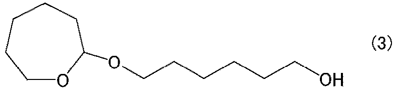

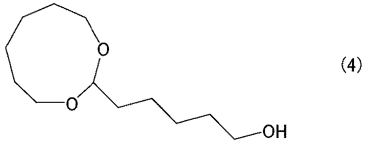

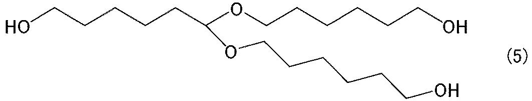

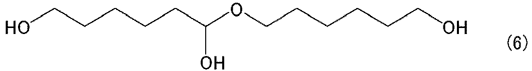

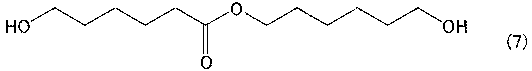

From the viewpoint of having significant influence on the reactivity of the 1,6-hexanediol composition and the reactivity of a polymer such as a polyester obtained by reacting the 1,6-hexanediol composition, the component (B) may be 6-hydroxyhexanal.

The total content of (B) 6-hydroxyhexanal and/or a derivative thereof (preferably, the content of 6-hydroxyhexanal) with respect to the total amount of the 1,6-hexanediol composition of the present invention is 1500 ppm by mass or less; however, the total content is preferably 1000 ppm by mass or less, more preferably 500 ppm by mass or less, even more preferably 200 ppm by mass or less, and particularly preferably 0 ppm by mass (not included). This tends to result in satisfactory reactivity of the composition of the present invention, satisfactory reactivity of the polymer (polyester or the like) produced using the composition of the present invention as a reaction raw material, and satisfactory tensile strength and tensile elongation of polyurethane. Furthermore, coloring or odor of the composition or the polymer produced using the composition as a reaction raw material can be reduced.

In the present specification, the total content of (B) 6-hydroxyhexanal and/or a derivative thereof is a value measured by gas chromatography mass spectrometry (GC/MS).

As described above, when the contents of the component (A) and the component (B) are in the above-described ranges, the composition of the present invention has excellent reactivity. Furthermore, a polymer such as a polyester produced using the composition of the present invention as a reaction raw material also has excellent reactivity. The reason why such operating effects are exhibited is not clearly understood, but is speculated as follows.

Since a secondary hydroxyl group has low reactivity, when a large amount of the component (A) is included in the composition of the present invention, a polymer such as a polyester produced using the composition of the present invention as a reaction raw material may often have secondary hydroxyl groups at the ends, and the reactivity of the polymer such as a polyester is decreased. Furthermore, when a large amount of the component (B) is included in the composition of the present invention, chain termination or branching occurs at the time of performing a polymerization reaction using the composition of the present invention as a reaction raw material.

Therefore, it is speculated that by adjusting the total content of the component (A) to be equal to or less than a specific amount, and the total content of the component (B) to be equal to or less than a specific amount, excellent reactivity of the 1,6-hexanediol composition and excellent reactivity of the polymer such as a polyester obtained by reacting the 1,6-hexanediol composition are obtained.

Furthermore, a polyurethane produced using the composition of the present invention as a reaction raw material has satisfactory tensile strength and tensile elongation. This is because when a polyol (reaction raw material of polyurethane) is synthesized from the composition of the present invention, the production of a polyol having secondary hydroxyl group terminals is suppressed to a low level. Therefore, it is speculated that because a decrease in the molecular weight does not occur during polyurethane formation, and because the amount of hydroxyl groups at the polyester terminals increases, branching occurs during polyurethane formation, resulting in a decrease in the cohesive power of the polyurethane.

### <<Alkali metal element>>

It is preferable that the 1,6-hexanediol composition of the present invention further contains an alkali metal element.

Usually, when a polyester is heated in the presence of water, catalyst residues generated from a dehydration condensation reaction form coordinate bonds with the ester bond moieties of the polyester, causing a hydrolysis reaction of ester; however, the alkali metal element inhibits the catalyst residues from coordinating to the ester moiety of the polyester. This can favorably suppress the hydrolysis of a polyester and a polyurethane having ester bonds, and satisfactory hydrolysis resistance is obtained.

On the other hand, in a case where the composition contains a large amount of the alkali metal element, when a polymer such as a polyester is synthesized, there is a risk that the alkali metal element may form coordinate bonds with the catalyst and inhibit the inherent action of the catalyst, that is, the action of increasing the rate of the polymerization reaction. Thus, by incorporating only a specific amount of an alkali metal element into the 1,6-hexanediol composition of the present invention, excessive lowering of the inherent action of the catalyst due to the alkali metal can be suppressed, and therefore, there is no adverse effect even when a polymer such as a polyester or a polyurethane is synthesized.

Therefore, when the 1,6-hexanediol composition of the present invention further contains an alkali metal element and contains a specific amount of the alkali metal element, satisfactory hydrolysis resistance can be imparted to the polymer. **In** addition, even when a polymer such as a polyester or a polyurethane is synthesized by using the 1,6-hexanediol composition of the present invention as a reaction raw material, the 1,6-hexanediol composition does not have adverse effect, and a polymer obtained by reacting the 1,6-hexanediol composition (for example, a polyester, or a polyurethane having ester bonds) has satisfactory hydrolysis resistance.

Therefore, specifically, the total content of the alkali metal element with respect to the total amount of the 1,6-hexanediol composition of the present invention is preferably 0.1 to 1000 ppm by mass.

The alkali metal element is not particularly limited, and examples thereof include lithium, sodium, potassium, rubidium, and cesium. These may be used singly, or two or more kinds thereof may be used in combination. Among them, sodium and potassium are preferred.

The state of existence of the alkali metal element included in the composition of the present invention is not particularly limited, and may be a simple substance of an alkali metal, an alkali metal compound, an alkali metal ion, or the like.

Specific examples of the alkali metal compound include metal salts of the above-described alkali metal elements and acids such as acetic acid, phosphoric acid, and nitric acid.

The total content of the alkali metal element (preferably, the total content of either or both of sodium metal element and potassium metal element) with respect to the total amount of the 1,6-hexanediol composition of the present invention is 0.1 to 1000 ppm by mass, but is preferably 0.1 to 800 ppm by mass, and more preferably 0.1 to 600 ppm by mass. When the total content of the alkali metal is 0.1 ppm by mass or more, more satisfactory reactivity, hydrolysis resistance, tensile strength, and tensile elongation are obtained, and when the total content is 1000 ppm by mass or less, more satisfactory reactivity tends to be obtained at the time of synthesizing a polymer, for example, at the time of synthesizing a polyester or a polyurethane using the 1,6-hexanediol composition as a raw material.

**In** the present specification, the total content of the alkali metal is a value measured by inductively coupled plasma mass spectrometry (ICP-MS).

### <<Glycerin>>

The 1,6-hexanediol composition of the present invention may further contain glycerin, and the content of glycerin with respect to the total amount of the composition is preferably 2000 ppm by mass or less, more preferably 1500 ppm by mass or less, even more preferably 1000 ppm by mass or less, particularly preferably 500 ppm by mass or less, and most preferably 0 ppm by mass (not included). When the content of glycerin is 2000 ppm by mass or less, at the time of synthesizing a polymer such as a polyester by reacting the 1,6-hexanediol composition, glycerin being excessively incorporated into the polymer can be suppressed, and therefore, the occurrence of branching in the polymer such as a polyester and the occurrence of gelation can be suppressed more favorably, while satisfactory reactivity tends to be obtained.

In the present specification, the content of glycerin is a value measured by gas chromatography mass spectrometry (GC/MS).

The 1,6-hexanediol composition of the present invention may be produced such that the total content of the component (A) and the total content of the component (B) are in the above-described ranges.

Furthermore, when the content of the alkali metal in the 1,6-hexanediol composition of the present invention is small, for example, a metal salt of an acid such as acetic acid, phosphoric acid, or nitric acid and an alkali metal may be added thereto such that the total content of the alkali metal falls within the above-described range.

### <Method for producing 1,6-hexanediol composition>

As the 1,6-hexanediol composition of the present invention, specifically, a 1,6-hexanediol composition (1,6-hexanediol composition induced from a biomass resource) obtained by the following environmentally friendly production method (method for producing a 1,6-hexanediol composition of the present invention) can be favorably used.

The method for producing a 1,6-hexanediol composition of the present invention will be described.

The method for producing a 1,6-hexanediol composition of the present invention includes: a step (1) of producing a 1,6-hexanediol composition from 6-hydroxycaproic acid and/or a derivative thereof obtained from a biomass resource-derived raw material; and a step (2) of purifying the 1,6-hexanediol composition obtained in the step (1) by ion exchange and/or distillation.

### <<Step (1)>>

The step (1) is a step of producing a 1,6-hexanediol composition from 6-hydroxycaproic acid and/or a derivative thereof obtained from a biomass resource-derived raw material. Here, when it is said that a 1,6-hexanediol composition is produced from 6-hydroxycaproic acid and/or a derivative thereof obtained from a biomass resource-derived raw material, it means that a 1,6-hexanediol composition is produced from a biomass resource-derived raw material via 6-hydroxycaproic acid and/or a derivative thereof. The phrase "via 6-hydroxycaproic acid and/or a derivative thereof" means that a process may involve 6-hydroxycaproic acid and/or a derivative thereof, and the process may be, for example, a step of converting from a biomass resource-derived raw material into a 1,6-hexanediol composition through 6-hydroxycaproic acid and/or a derivative thereof within the cells of a microorganism; may be a step of producing 6-hydroxycaproic acid and/or a derivative thereof from a biomass resource-derived raw material by a microorganism, and converting the compound into a 1,6-hexanediol composition by the microorganism using the culture liquid containing the compound as it is; or may be a step of subjecting the culture liquid to purification, and converting the compound into a 1,6-hexanediol composition by a microorganism in a different reaction tank.

The derivative of 6-hydroxycaproic acid is not particularly limited as long as it is a derivative of 6-hydroxycaproic acid, and examples thereof include 6-hydroxy-hexanoyl-CoA, which is a thioester of 6-hydroxycaproic acid, a cyclization product of 6-hydroxycaproic acid, a condensation product of 6-hydroxycaproic acid with itself, and an ester compound such as a condensation product of 6-hydroxycaproic acid and 1,6-hexanediol. These may be used singly, or two or more kinds thereof may be used in combination. Among them, 6-hydroxy-hexanoyl-CoA, which is a thioester of 6-hydroxycaproic acid, is preferred.

In the present specification, CoA means coenzyme A.

As the 6-hydroxycaproic acid and/or a derivative thereof, 6-hydroxycaproic acid and 6-hydroxy-hexanoyl-CoA are preferred, and 6-hydroxycaproic acid is more preferred.

It is preferable that the step (1) is a step of producing a 1,6-hexanediol composition under the conditions at 70°C or lower, and more preferably a step of producing a 1,6-hexanediol composition under the conditions at 50°C or lower. The lower limit of the temperature is not particularly limited, but is preferably 20°C or higher. By carrying out the production at a relatively low temperature, it is possible to reduce carbon dioxide emission during the production, side reactions such as cyclization accompanied by dehydration can be reduced, and generation of by-products such as a cyclic oligomer can be suppressed. This also leads to the suppression of fogging, which is a problem in automotive interior materials and the like. In addition, factors inhibiting the packing of polymer chains are reduced, and it is possible to enhance the film physical properties.

The step (1) may be carried out, for example, using a microorganism capable of producing (biosynthesizing) 1,6-hexanediol. Examples of the microorganism capable of producing 1,6-hexanediol include the microorganisms described in, for example, JP 2020-114227 A and JP 6680671 B2. These may be used singly, or two or more kinds thereof may be used in combination. Those ordinarily skilled in the art can easily produce or obtain microorganisms capable of producing 1,6-hexanediol based on known technologies such as those mentioned in the above-described patent publications.

3-Oxopropionate and 3-hydroxypropanal are produced by the metabolic pathways in microorganisms.

An example of the production of 3-hydroxypropanal in a microorganism will be described. As described in FIG. 1 of JP 6680671 B2 and the like, in microorganisms, 3-phosphoglyceraldehyde is produced from sugars (pentoses and hexoses) through a glycolytic metabolic pathway such as a pentose phosphate pathway (PP pathway). Similarly, in microorganisms, 3-phosphoglyceraldehyde is produced from glycerin by a metabolic pathway. Then, 3-hydroxypropanal is produced from 3-phosphoglyceraldehyde through reactions catalyzed by a plurality of enzymes carried by microorganisms. Furthermore, 3-hydroxypropanal is produced from glycerin in a single reaction by diol dehydratase and/or glycerol dehydratase.

An example of the production of 3-oxopropionate by microorganisms will be described. As described in FIG. 1 of JP 6680671 B2 and the like, in microorganisms, 3-phosphoglyceric acid and 2-phosphoglyceric acid are produced from sugars (pentoses and hexoses) through a glycolytic metabolic pathway such as the Embden-Meyerhof pathway (EM pathway). Glyceric acid is produced from 3-phosphoglyceric acid and 2-phosphoglyceric acid by utilizing the enzymes described in Table 1 of JP 6680671 B2 and the like, or a reverse reaction of glyceric acid kinase. 3-Oxopropionate is produced from glyceric acid by diol dehydratase and/or glycerol dehydratase. In addition, 3-oxopropionate can also be produced from oxaloacetic acid, which is a metabolic intermediate in the TCA cycle, in a single reaction by keto acid decarboxylase. Oxaloacetic acid can also be produced from sugars by phosphoenolpyruvate carboxylase and pyruvate carboxylase without going through the TCA cycle.

1,6-Hexanediol is produced from 3-oxopropionate and 3-hydroxypropanal, which are produced by metabolic pathways in microorganisms, by utilizing various pathways shown in, for example, FIG. 2 to FIG. 5 of JP 6680671 B2.

Suitable examples of a microorganism capable of producing 1,6-hexanediol from 3-oxopropionate and 3-hydroxypropanal, that is, a microorganism capable of producing 1,6-hexanediol, include the following microorganisms.

Microorganisms having genes encoding ten enzymes of the 1,6-hexanediol pathway consisting of 4,6-dihydroxy-2-oxo-hexanoate aldolase (2A in the drawings of JP 6680671 B2), 4,6-dihydroxy-2-oxo-hexanoate 4-dehydratase (2B in the drawings of JP 6680671 B2), 6-hydroxy-3,4-dehydro-2-oxohexanoate 3-reductase (2C in the drawings of JP 6680671 B2), 6-hydroxy-2-oxohexanoate 2-reductase (2D in the drawings of JP 6680671 B2), 2,6-dihydroxy-hexanoate CoA-transferase (2E in the drawings of JP 6680671 B2), 2,6-dihydroxy-hexanoyl-CoA 2-dehydratase (2F in the drawings of JP 6680671 B2), 6-hydroxy-2,3-dehydrohexanoyl-CoA 2,3-reductase (2G in the drawings of JP 6680671 B2), 6-hydroxyhexanoyl-CoA-transferase (4F3 in the drawings of JP 6680671 B2), 6-hydroxyhexanoate 1-reductase (5R in the drawings of JP 6680671 B2), and 6-hydroxyhexanal 1-reductase (5S in the drawings of JP 6680671 B2).

The microorganisms having genes encoding the ten enzymes of the 1,6-hexanediol pathway are not particularly limited as long as they have the genes encoding the ten enzymes of the 1,6-hexanediol pathway, and examples thereof include prokaryotes and eukaryotes.

Furthermore, the microorganisms having the genes encoding the ten enzymes of the 1,6-hexanediol pathway may be genetically engineered microorganisms into which all and/or some of the above-describes genes (enzymes) have been introduced so as to have the genes encoding the ten enzymes of the 1,6-hexanediol pathway. For example, when a microorganism to be used has only eight enzymes among the above-described ten enzymes, genes encoding the remaining two enzymes may be introduced into the microorganism. Furthermore, when a microorganism to be used has only some enzymes among the above-described ten enzymes, a single microorganism or a plurality of microorganisms having genes encoding the remaining enzymes may be used in combination.

In addition, it is preferable that the microorganisms have genes encoding diol dehydratase and/or glycerol dehydratase. Here, introduction of the genes can be carried out by known techniques.

Examples of the prokaryotes include bacteria.

Examples of the eukaryotes include yeasts and filamentous fungi.

Examples of the bacteria include bacteria belonging to the family Enterobacteriaceae, coryneform bacteria, bacteria of the genus Bacillus, acetic acid bacteria, actinomycetes, and lactic acid bacteria.

Examples of the bacteria belonging to the family Enterobacteriaceae include bacteria belonging to the genus Escherichia, the genus Enterobacter, the genus Pantoea, the genus Klebsiella, the genus Serratia, the genus Erwinia, the genus Photorhabdus, the genus Providencia, the genus Salmonella, and the genus Morganella. Specifically, the bacteria classified into the family Enterobacteriaceae according to the classification method used in the database of the National Center for Biotechnology Information (NCBI) (http://www.ncbi.nlm.nih.gov/Taxonomy/Browser/wwwtax.cgi?id=91347) can be used.

Examples of the bacteria of the genus Escherichia include, but are not particularly limited to, bacteria classified into the genus Escherichia by classification known to the experts in microbiology. Examples of the bacteria of the genus Escherichia include those described in the book written by Neidhardt et al. (Backmann, B.J. 1996. Derivations and Genotypes of some mutant derivatives of Escherichia coli K-12, p. 2460-2488. Table 1. In F.D. Neidhardt (ed.), Escherichia coli and Salmonella Cellular and Molecular Biology/Second Edition, American Society for Microbiology Press, Washington, D.C.). Examples of the bacteria of the genus Escherichia include Escherichia coli. Examples of Escherichia coli include Escherichia coli stain K-12 such as strain W3110 (ATCC 27325) and strain MG1655 (ATCC 47076); Escherichia coli strain K5 (ATCC 23506); Escherichia coli strain B such as strain BL21 (DE3); and derivative strains thereof.

Examples of the bacteria of the genus Enterobacter include Enterobacter agglomerans and Enterobacter aerogenes. Examples of the bacteria of the genus Pantoea include Pantoea ananatis, Pantoea stewartii, Pantoea agglomerans, and Pantoea citrea. Examples of the bacteria of the genus Erwinia include Erwinia amylovora and Erwinia carotovora. Examples of the bacteria of the genus Klebsiella include Klebsiella planticola.

Examples of the coryneform bacteria include bacteria belonging to the genus Corynebacterium, the genus Brevibacterium, and the genus Microbacterium.

Specific examples of the coryneform bacteria include the following species:
Corynebacterium acetoacidophilum
Corynebacterium acetoglutamicum
Corynebacterium alkanolyticum
Corynebacterium callunae
Corynebacterium crenatum
Corynebacterium glutamicum
Corynebacterium lilium
Corynebacterium melassecola
Corynebacterium thermoaminogenes (Corynebacterium efficiens)
Corynebacterium herculis
Brevibacterium divaricatum (Corynebacterium glutamicum)
Brevibacterium flavum (Corynebacterium glutamicum)
Brevibacterium immariophilum
Brevibacterium lactofermentum (Corynebacterium glutamicum)
Brevibacterium roseum
Brevibacterium saccharolyticum
Brevibacterium thiogenitalis
Corynebacterium ammoniagenes (Corynebacterium stationis)
Brevibacterium album
Brevibacterium cerinum
Microbacterium ammoniaphilum

Specific examples of the Coryneform bacteria include the following strains:
Corynebacterium acetoacidophilum ATCC 13870
Corynebacterium acetoglutamicum ATCC 15806
Corynebacterium alkanolyticum ATCC 21511
Corynebacterium callunae ATCC 15991
Corynebacterium crenatum AS1.542
Corynebacterium glutamicum ATCC 13020, ATCC 13032, ATCC 13060, ATCC 13869, FERM BP-734
Corynebacterium lilium ATCC 15990
Corynebacterium melassecola ATCC 17965
Corynebacterium efficiens (Corynebacterium thermoaminogenes) AJ12340 (FERM BP-1539) Corynebacterium herculis ATCC 13868
Brevibacterium divaricatum (Corynebacterium glutamicum) ATCC 14020
Brevibacterium flavum (Corynebacterium glutamicum) ATCC 13826, ATCC 14067, AJ12418 (FERM BP-2205)
Brevibacterium immariophilum ATCC 14068
Brevibacterium lactofermenum (Corynebacterium glutamicum) ATCC 13869
Brevibacterium roseum ATCC 13825
Brevibacterium sacccharolyticum ATCC 14066
Brevibacterium thiogenitalis ATCC 19240
Corynebacterium ammoniagenes (Corynebacterium stationis) ATCC 6871, ATCC 6872 Brevibacterium album ATCC 15111
Brevibacterium cerinum ATCC 15112
Microbacterium ammoniaphilum ATCC 15354

Incidentally, the bacteria of the genus Corynebacterium have been conventionally classified into the genus Brevibacterium; however, bacteria integrated into the genus Corynebacterium (Int. J. Syst. Bacteriol., 41, 255 (1991)) are also included therein. Furthermore, Corynebacterium stationis also includes bacteria that have been conventionally classified into Corynebacterium ammoniagenes but have been reclassified into Corynebacterium stationis based on the analysis of 16S rRNA base sequence and the like (Int. J. Syst. Evol. Microbiol., 60, 874-879 (2010)).

Examples of the bacteria of the genus Bacillus include Bacillus subtilis, Bacillus amyloliquefaciens, Bacillus pumilus, Bacillus licheniformis, Bacillus megaterium, Bacillus brevis, Bacillus polymixa, and Bacillus stearothermophilus. Specific examples of Bacillus subtilis include Bacillus subtilis strain 168 Marburg (ATCC 6051) and Bacillus subtilis strain PY79 (Plasmid, 1984, 12, 1-9). Specific examples of Bacillus amyloliquefaciens include Bacillus amyloliquefaciens strain T (ATCC 23842) and Bacillus amyloliquefaciens strain N (ATCC 23845).

Examples of the acetic acid bacteria include bacteria belonging to the genus Gluconobacter, the genus Acetobacter, the genus Gluconacetobacter, the genus Acidicaldus, the genus Acidiphilium, the genus Acidisphaera, the genus Acidocella, the genus Acidomonas, the genus Asaia, the genus Belnapia, the genus Craurococcus, the genus Granulibacter, the genus Kozakia, the genus Leahibacter, the genus Muricoccus, the genus Neoasaia, the genus Oleomonas, the genus Paracraurococcus, the genus Rhodopila, the genus Roseococcus, the genus Rubritepida, the genus Saccharibacter, the genus Stella, the genus Swaminathania, the genus Teichococcus, and the genus Zavarzinia.

Specific examples of the acetic acid bacteria include, for example, Gluconobacter oxydans, Acetobacter xylinum, Asidomonas methanolicus, Asaia bogorensis, Asaia krungthepensis, Belnapia moabensis, Gluconacetobacter xylinus, Granulibacter bethesdensis, Kozakia baliensis, and Oleomonas sagaranensis.

Examples of the Actinomycetes include bacteria belonging to the genus Actinomyces, the genus Mycobacterium, the genus Nocardia, the genus Streptomyces, the genus Actinoplanes, and the genus Rhodococcus.

Specific examples of the Actinomycetes include Streptomyces coelicolor, Streptomyces griseus, Streptomyces avermitilis, and Rhodococcus zopfii.

Examples of the lactic acid bacteria include bacteria belonging to the genus Lacticaseibacillus, the genus Lactobacillus, the genus Ligilactobacillus, the genus Limosilactobacillus, the genus Liquorilactobacillus, the genus Lactiplantibacillus, the genus Streptococcus, the genus Lactococcus, and the genus Enterococcus.

Specific examples of the lactic acid bacteria include, for example, Lacticaseibacillus casei, Lactobacillus gasseri, Lactobacillus acidophilus, Lactobacillus helveticus, Lactobacillus delbrueckii subsp. bulgaricus, Lactobacillus delbrueckii subsp. delbrueckii, Lactobacillus johnsonii, Ligilactobacillus salivarius, Limosilactobacillus fermentum, Liquorilactobacillus mali, Lactiplantibacillus plantarum, Streptococcus thermophilus, Lactococcus lactis subsp. lactis, Lactococcus lactis subsp. cremoris, Lactococcus plantarum, Lactococcus raffinolactis, Lactococcus cremoris, Enterococcus faecalis, and Enterococcus faecium.

Examples of bacteria other than the above-described bacteria include bacteria belonging to the genus Pseudomonas, the genus Agrobacterium, the genus Alicyclobacillus, the genus Arthrobacter, the genus Azotobacter, the genus Chromatium, the genus Methylobacterium, the genus Rhodobacter, the genus Rhodopseudomonas, the genus Rhodospirillum, the genus Zymomonas, the genus Achromobacter, the genus Aerobacter, the genus Alcaligenes, the genus Arthrobacter, the genus Erwinia, the genus Microbacterium, the genus Micrococcus, the genus Protaminobacter, the genus Proteus, the genus Sartina, the genus Xanthomonas, the genus Aeromonas, the genus Flavobacterium, and the genus Rhizobium.

Examples of the yeasts include yeasts belonging to the genus Saccharomyces, the genus Candida, the genus Phichia, the genus Schizosaccharomyces, the genus Kluyveromyces, the genus Rhodotorula, the genus Cryptococcus, the genus Torulopsis, the genus Hansenula, the genus Issatchenkia, the genus Kluyveromyces, and the genus Yarrowia.

Specific examples of the yeasts include, for example, Saccharomyces cerevisiae, Candida utilis, Pichia pastoris, Hansenula polymorpha, and Schizosaccharomyces pombe.

Examples of the filamentous fungi (mold) include fungi belonging to the genus Aspergillus, the genus Paecilomyces, the genus Penicillium, the genus Neurospora, the genus Trichoderma, the genus Fusarium, and the genus Chrysosporium.

Specific examples of the filamentous fungi (mold) include, for example, Aspergillus oryzae, Paecilomyces saturatus, Paecilomyces divaricatus, and Penicillium camemberti (P. camemberti).

These strains can be furnished from, for example, the American Type Culture Collection (address: 12301 Parklawn Drive, Rockville, Maryland 20852 P.O. Box 1549, Manassas VA 20108, United States of America). That is, a registration number corresponding to each strain is assigned, and each strain can be obtained by utilizing this registration number (see http://www.atcc.org/). The registration number corresponding to each strain is described in the catalogue of the American Type Culture Collection. Furthermore, these strains can be obtained from the depository institution at which each strain has been deposited.

The above-described microorganisms may be used singly, or two or more kinds thereof may be used in combination. Among them, prokaryotes are preferred, bacteria are more preferred, microorganisms belonging to the family Enterobacteriaceae are even more preferred, microorganisms belonging to the genus Escherichia are particularly preferred, and Escherichia coli is most preferred. That is, prokaryotes having genes encoding the ten enzymes of the 1,6-hexanediol pathway are preferred, bacteria having genes encoding the ten enzymes of the 1,6-hexanediol pathway are more preferred, microorganisms belonging to the family Enterobacteriaceae having genes encoding the ten enzymes of the 1,6-hexanediol pathway are even more preferred, microorganisms belonging to the genus Escherichia having genes encoding the ten enzymes of the 1,6-hexanediol pathway are particularly preferred, and Escherichia coli having genes encoding the ten enzymes of the 1,6-hexanediol pathway is most preferred.

In a case where the genes (enzymes) are exogenously introduced into a microorganism and expressed, it is preferable that the 4,6-dihydroxy-2-oxo-hexanoate aldolase is 2,4-dihydroxyhept-2-ene-1,7-dioate aldolase encoded by HpaI gene of Escherichia coli, the 4,6-dihydroxy-2-oxo-hexanoate 4-dehydratase is 2-oxo-hept-4-ene-1,7-dioate hydratase encoded by HpcG/HpaH genes of Escherichia coli, the 6-hydroxy-3,4-dehydro-2-oxohexanoate 3-reductase is NADP-dependent alkenal reductase P1 of Arabidopsis thaliana available under GenBank Accession Number CAC01710.1, the 6-hydroxy-2-oxohexanoate 2-reductase is D-2-hydroxy acid dehydrogenase encoded by panE gene of Lactococcus lactis, the 2,6-dihydroxy-hexanoate CoA-transferase and the 6-hydroxyhexanoyl-CoA-transferase are glutaconate-CoA-transferases encoded by HadA gene of Clostridium difficile, the 2,6-dihydroxy-hexanoyl-CoA 2-dehydratase is 2-hydroxyisocaproyl-CoA dehydratase expressed by Clostridium difficile, the 6-hydroxy-2,3-dehydro-hexanoyl-CoA 2,3-reductase is trans-2-enoyl CoA reductase of Treponema denticola available under the GenBank Accession Number AE017248, the 6-hydroxyhexanoate 1-reductase is ATP/NADPH-CAR of Nocardia iowensis available under the GenBank Accession Number AAR91681.1, and/or the 6-hydroxyhexanal 1-reductase is 6-hydroxyhexanoate dehydrogenase of Rhodococcus available under the GenBank Accession Number AAN37489.1.

By using the above-described microorganisms, it is possible to carry out (a) a step of converting C₃ aldehyde and pyruvic acid into a C₆ β-hydroxyketone intermediate through aldol addition; and subsequently (b) a step of converting the C₆ β-hydroxyketone intermediate into 1,6-hexanediol or a solvate thereof through an enzymatic step. Here, it is preferable that the conversion includes reduction of enoyl or enoate, reduction of a ketone, reduction of an aldehyde, dehydration, formation of a thioester, reduction of a thioester, or a combination thereof.

Furthermore, it is preferable that a step of preparing C₃ aldehyde and pyruvic acid from a supply source selected from glycerin, pentoses, hexoses, phosphoglycerates, other carbon sources, intermediates of the glycolytic pathway, intermediates of propanoic acid metabolism, or combinations thereof is further included.

Furthermore, C₃ aldehyde is obtained through a series of enzymatic steps, and here, it is preferable that the enzymatic steps include dehydration of a diol.

More specifically, by using the above-described microorganisms, 1,6-hexanediol is produced from 3-oxopropionate and 3-hydroxypropanal produced by the metabolic pathways in the microorganisms through the pathways 2A, 2B, 2C, 2D, 2E, 2F, 2G, 4F3, 5R, and 5S shown in FIG. 2 and FIG. 5 of JP 6680671 B2. In this biosynthesis pathway, 6-hydroxy-hexanoyl-CoA is produced as the enzymatic reactions of pathways up to 2G proceed, subsequently 6-hydroxycaproic acid is produced as the enzymatic reaction of 4F3 proceeds, subsequently 6-hydroxyhexanal is produced as the enzymatic reaction of 5R proceeds, and subsequently 1,6-hexanediol is produced as the enzymatic reaction of 5S proceeds.

Therefore, it is possible to carry out the step (1) by culturing the above-described microorganism in a medium including a biomass resource-derived raw material. That is, by culturing the above-described microorganism in a medium including a biomass resource-derived raw material, a 1,6-hexanediol composition can be produced from 6-hydroxycaproic acid and/or a derivative thereof obtained from the biomass resource-derived raw material.

The biomass resource-derived raw material is not particularly limited, and examples thereof include carbon sources used in medium components, such as monosaccharides including pentoses such as xylose, xylulose, ribulose, arabinose, lyxose, and ribose, and hexoses such as allose, altrose, glucose, mannose, gulose, idose, talose, galactose, fructose, psicose, sorbose, and tagatose, disaccharides such as lactose, cellobiose, sucrose, and maltose, polysaccharides such as starch, cellulose, agarose, and dextran, alcohols such as sorbitol, ethanol, and glycerin; peptone, tryptone, and casamino acids; and nitrogen sources used in medium components, such as organic nitrogen compounds such as peptone, tryptone, casamino acids, yeast extract, meat extract, and corn steep liquor. These may be used singly, or two or more kinds thereof may be used in combination. Among them, carbon sources used in medium components are preferred, and a monosaccharide and an alcohol are more preferred. As the monosaccharide, a hexose is even more preferred, and glucose is particularly preferred. As the alcohol, glycerin is even more preferred.

Therefore, glucose and glycerin are most preferred as the biomass resource-derived raw material.

The medium is not particularly limited as long as it includes the biomass resource-derived raw material, and the medium may be a conventional medium including a carbon source, a nitrogen source, inorganic ions, and an organic nutrient source as necessary, or can be prepared as appropriate depending on the microorganism used. Among them, it is preferable that the medium contains the above-described carbon source as a biomass resource-derived raw material.

Carbon sources other than the above-described components are not particularly limited as long as they can be utilized by microorganisms, and examples thereof include organic acids such as fumaric acid, citric acid, acetic acid, and propionic acid, salts thereof, and carbohydrates such as paraffin. These may be used singly, or two or more kinds thereof may be used in combination.

Examples of nitrogen sources other than the above-described components include ammonium salts of inorganic acids, such as ammonium sulfate and ammonium chloride, ammonium salts of organic acids, such as ammonium fumarate and ammonium citrate; and nitric acid salts such as sodium nitrate and potassium nitrate. These may be used singly, or two or more kinds thereof may be used in combination.

For the medium, nutrients that are used in ordinary media, such as trace amounts of metal salts, vitamins, and hormones, may be used. These may be used singly, or two or more kinds thereof may be used in combination. Among them, it is preferable that the medium contains sodium and/or potassium as the trace amount metal salt.

The culture conditions are not particularly limited, and for example, culture may be carried out for about 4 to 140 hours while appropriately controlling the pH and temperature in the range of pH 3 to 11 and the temperature range of 20°C to 70°C (more preferably 20°C to 50°C). Culture may be carried out under aerobic conditions or anaerobic conditions, and may be appropriately selected in accordance with the microorganism used; however, aerobic conditions are preferred.

As described above, a 1,6-hexanediol composition can be produced by culturing the above-described microorganism in a medium containing a biomass resource-derived raw material. Since the 1,6-hexanediol composition is present in the medium together with microorganisms, the microorganisms may be removed from the medium as necessary. The method for removing the microorganisms is not particularly limited, and may be performed by, for example, centrifugation and/or membrane separation.

Centrifugation is not particularly limited, and for example, continuous centrifugation by a continuous centrifuge can be used. Examples of the continuous centrifuge include a basket type centrifuge, a disk type centrifuge, and a nozzle type centrifuge. These may be used singly, or two or more kinds thereof may be used in combination.

The membrane used for membrane separation is not particularly limited and may be, for example, a membrane having a pore size of 10.0 µm or less, and specific examples include a microfiltration membrane, an ultrafiltration membrane, a nanofiltration membrane, and a reverse osmosis membrane. These may be used singly, or two or more kinds thereof may be used in combination. Furthermore, the shape of the membrane is not particularly limited, and may be any shape such as a flat membrane, a hollow fiber membrane, a spiral membrane, a tubular membrane, or a pleated membrane. The type of filtration is also not particularly limited, and either the dead-end mode or the tangential flow mode can be applied; however, the tangential flow mode is preferred.

### <<Step (2)>>

Step (2) is a step of purifying the 1,6-hexanediol composition obtained in step (1), by ion exchange and/or distillation. The step (2) is not particularly limited as long as it is a step of appropriately removing the impurifies included in the 1,6-hexanediol composition obtained in the step (1), for example, the medium containing the 1,6-hexanediol composition after removal of microorganisms, by ion exchange and/or distillation; however, it is preferable that the step (2) is a step of purifying the 1,6-hexanediol composition obtained in the step (1), by ion exchange and distillation. Furthermore, the ion exchange is preferably cation exchange and anion exchange, and it is more preferable to perform cation exchange and anion exchange in this order. Furthermore, regarding the distillation, it is preferable to carry out the removal of water, the removal of components having lower boiling points than 1,6-hexanediol, and the removal of components having higher boiling points than 1,6-hexanediol, and it is more preferable to carry out the removal of water, the removal of components having lower boiling points than 1,6-hexanediol, and the removal of components having higher boiling points than 1,6-hexanediol, in this order.

The 1,6-hexanediol composition that has been obtained in the step (1) and is subjected to the step (2) is not particularly limited as long as it is a 1,6-hexanediol composition obtained in the step (1); however, the 1,6-hexanediol composition is preferably a liquid obtained by removing microorganisms from the medium obtained in the step (1) and containing the 1,6-hexanediol composition, and more preferably a liquid obtained by removing microorganisms by subjecting the medium obtained in the step (1) and containing the 1,6-hexanediol composition to centrifugation and membrane separation treatments.

An example of the step (2) will be described in detail below.

### [Ion exchange step]

In an ion exchange step, a 1,6-hexanediol composition is obtained through the following steps (a) and (b) in this order.
Step (a): A step of bringing the 1,6-hexanediol composition obtained in the step (1) into contact with a cation exchange resin to obtain a 1,6-hexanediol composition A.
Step (b): A step of bringing the 1,6-hexanediol composition A obtained in step (a) into contact with an anion exchange resin to obtain a 1,6-hexanediol composition B.

Examples of the components to be removed by contact with a cation exchange resin in the step (a) include metal cations and ammonium ion.

Examples of the components to be removed by contact with an anion exchange resin in the step (b) include chloride ion, sulfate ion, phosphate ion, and organic acids. The organic acids mentioned herein include (A) a compound having two or more secondary hydroxyl groups in one molecule having an acid group.

The treatment using ion exchange resins is not particularly limited; however, it is preferable to carry out the treatment in a batch manner or in a column manner.

### <Step (a): Step of removing cations included in 1,6-hexanediol composition>

**In** step (a), cations are removed by bringing the 1,6-hexanediol composition into contact with a cation exchange resin.

The cation exchange resin used may be, for example, a strongly acidic resin or a weakly acidic resin; however, there is no particular limitation. Furthermore, examples thereof include, but are not particularly limited to, styrene-based, acrylic, and hydrogel-based cation exchange resins. Furthermore, examples thereof include, but are not particularly limited to, gel type, porous type, and highly porous type cation exchange resins. Examples of the form of the resin include, but are not limited to, a powdered form, a spherical form, a fibrous form, and a membranous form.

### <Step (b): Step of removing anions included in 1,6-hexanediol composition>

In step (b), anions are removed by bringing the 1,6-hexanediol composition into contact with an anion exchange resin.

The anion exchange resin used may be, for example, a strongly basic resin or a weakly basic resin; however, there is no particular limitation. Furthermore, examples thereof include, but are not particularly limited to, styrene-based, acrylic, and hydrogel-based anion exchange resins. Furthermore, examples thereof include, but are not particularly limited to, gel type, porous type, and highly porous type anion exchange resins. Examples of the form of the resin include, but are not limited to, a powdered form, a spherical form, a fibrous form, and a membranous form.

Incidentally, in the above description, a method of carrying out the step (a) and the step (b) in this order has been described; however, the step (b) and the step (a) may be carried out in this order. That is, the 1,6-hexanediol composition obtained in the step (1) may be brought into contact with an anion exchange resin and then brought into contact with a cation exchange resin.

Furthermore, the step (a) and the step (b) may be carried out at the same time. That is, the 1,6-hexanediol composition obtained in step (1) may be simultaneously brought into contact with a cation exchange resin and an anion exchange resin.

### [Distillation step]

Distillation is carried out through the following steps (c), (d), and (e) in this order, and a 1,6-hexanediol composition E is obtained. When it is desired to further increase the purity, the composition is optionally purified in step (f).
Step (c): A step of removing water included in a liquid containing the 1,6-hexanediol composition from the 1,6-hexanediol composition obtained in the above-described ion exchange step (for example, 1,6-hexanediol composition B) and obtaining a 1,6-hexanediol composition C.
Step (d): A step of removing components having lower boiling points than 1,6-hexanediol, from the 1,6-hexanediol composition C obtained in step (c) and obtaining a 1,6-hexanediol composition D.
Step (e): A step of removing components having higher boiling points than 1,6-hexanediol from the 1,6-hexanediol composition D obtained in step (d), and obtaining a 1,6-hexanediol composition E.
Step (f): A step of distilling the 1,6-hexanediol composition E obtained in step (e), and obtaining 1,6-hexanediol with higher purity.

Examples of the components having lower boiling points than 1,6-hexanediol in the step (d) include 1,3-propanediol, (A) the compound having two or more secondary hydroxyl groups in one molecule, and (B) 6-hydroxyhexanal and/or a derivative thereof.

Examples of the components having higher boiling points than 1,6-hexanediol in the step (e) include glycerin, (A) the compound having two or more secondary hydroxyl groups in one molecule, and (B) 6-hydroxyhexanal and/or a derivative thereof.

The distillation method is not particularly limited; however, it is preferable to remove the components by continuous or batch distillation.

### <Step (c): Step of removing water included in 1,6-hexanediol composition>

**In** step (c), water is removed from the 1,6-hexanediol composition B by heating the 1,6-hexanediol composition obtained in the above-described ion exchange step (for example, 1,6-hexanediol composition B) to a temperature at which water evaporates, and reducing pressure as necessary.

Examples of the apparatus to be used include, but are not particularly limited to, a continuous distillation column, a multiple effect evaporator, a thin film evaporator, an evaporator, a batch still, and an atomization separation apparatus.

### <Step (d): Step of removing components having lower boiling points than 1,6-hexanediol>

**In** step (d), components having lower boiling points than 1,6-hexanediol are removed from the 1,6-hexanediol composition C.

The step (d) is a step carried out for the purpose of both sufficiently removing components having lower boiling points in order to obtain 1,6-hexanediol with high purity and carrying out removal of trace amounts of components causative of coloring. **In** this operation, particularly components themselves causative of coloring, and components having lower boiling points than 1,6-hexanediol, such as hydrogenation products of the components causative of coloring, are removed or reduced.

The distillation in the step (d) can be carried out using, for example, known means and apparatuses such as atmospheric distillation, distillation under reduced pressure, and pressure distillation. Examples of the apparatus for removal include, but are not particularly limited to, a continuous distillation column, a multiple effect evaporator, a thin film evaporator, an evaporator, a batch still, and an atomization separation apparatus. The operation conditions used in the step (d) may be appropriately set by taking the composition of the 1,6-hexanediol composition C, the purity to be finally obtained, and the like into consideration, and are not particularly limited.

### <Step (e): Step of removing components having higher boiling points than 1,6-hexanediol>

In step (e), components having higher boiling points than 1,6-hexanediol are removed from the 1,6-hexanediol composition D obtained in step (d).

In the step (e), components having higher boiling points than 1,6-hexanediol, which are characteristic of fermentation methods, such as nitrogen-containing components, particularly derived from amino acids and proteins, and sugars and decomposition products thereof, are removed.

Examples of the apparatus for removal include, but are not limited to, a continuous distillation column, a multiple effect evaporator, a thin film evaporator, an evaporator, a batch still, and an atomization separation apparatus. The operation conditions used in the step (e) may be appropriately set by taking the composition of the 1,6-hexanediol composition D, the purity to be finally obtained, and the like into consideration, and are not particularly limited.

### <Step (f): Step of obtaining 1,6-hexanediol composition with higher purity>

In step (f), the 1,6-hexanediol composition E obtained through the steps (c), (d), and (e) is purified to obtain a 1,6-hexanediol composition with higher purity.

Examples of the apparatus for obtaining a 1,6-hexanediol composition with higher purity include, but are not limited to, a continuous distillation column, a multiple effect evaporator, a thin film evaporator, an evaporator, a batch still, and an atomization separation apparatus.

The operation conditions used in the step (f) may be appropriately set by taking the composition of the liquid to be purified, the purity to be finally obtained, and the like into consideration, and are not particularly limited.

By carrying out the step (e) and, if necessary, the step (f), the 1,6-hexanediol composition of the present invention is obtained.

In the above description, a method of carrying out the step (c), the step (d), and the step (e) in this order has been described; however, the steps may be carried out in a different order.

The method for producing a 1,6-hexanediol composition of the present invention may include step (1) and step (2), or may include steps other than the step (1) and the step (2), in addition to the step (1) and the step (2).

By carrying out the step (2), the total content of (A) the compound having two or more secondary hydroxyl groups in one molecule, and the total content of (B) 6-hydroxyhexanal and/or a derivative thereof will be within the above-described ranges; however, the step (2) may be repeatedly carried out so that the total content of (A) the compound having two or more secondary hydroxyl groups in one molecule and the total content of (B) 6-hydroxyhexanal and/or a derivative thereof are more suitably within the above-described ranges. By repeating the step (2), it is possible to further reduce the total content of (A) the compound having two or more secondary hydroxyl groups in one molecule and the total content of (B) 6-hydroxyhexanal and/or a derivative thereof.

### <Polymer>

The polymer of the present invention is a polymer obtained by reacting the 1,6-hexanediol composition of the present invention. The polymer is not particularly limited as long as it is a polymer having a structural unit derived from the 1,6-hexanediol composition of the present invention, and also includes an oligomer. Examples of the polymer of the present invention include a polyester, a polyurethane, a polycarbonate, a polyether, an epoxy-based polymer produced using the above-described epoxy group-containing 1,6-hexanediol derivative as a raw material, and an acrylic polymer produced using the above-described (meth)acryloyl group-containing 1,6-hexanediol derivative as a raw material. These may be used singly, or two or more kinds thereof may be used in combination. Among them, a polyester, a polyurethane, a polycarbonate, and a polyether are preferred, a polyester, a polyurethane, and a polyether are more preferred, and a polyester and a polyurethane are even more preferred.

Incidentally, the polymer of the present invention has at least a structural unit derived from the 1,6-hexanediol composition of the present invention, and also optionally contains a structural unit derived from the glycerin included in the 1,6-hexanediol composition.

The content of the structural unit derived from the 1,6-hexanediol composition of the present invention in 100% by mass of the polymer of the present invention is preferably 10% to 100% by mass, and more preferably 20% to 100% by mass. This tends to allow the effects to be obtained more favorably.

**In** the present specification, the content of each structural unit in the polymer is measured by NMR.

The total content of a component (A)-derived structural unit in one molecule in 100% by mass of the polymer of the present invention is preferably 50 ppm by mass or less, more preferably 10 ppm by mass or less, even more preferably 5 ppm by mass or less, and particularly preferably 0 ppm by mass (not included).

This tends to allow the effects to be obtained more favorably.

The total content of a component (B)-derived structural unit in 100% by mass of the polymer of the present invention is preferably 1000 ppm by mass or less, more preferably 500 ppm by mass or less, even more preferably 200 ppm by mass or less, and particularly preferably 0 ppm by mass (not included).

This tends to allow the effects to be obtained more favorably.

In the polymer of the present invention, the total content of the alkali metal elements (preferably, the total content of either or both of sodium metal element and potassium metal element) is preferably 0.05 to 500 ppm by mass, more preferably 0.05 to 400 ppm by mass, and even more preferably 0.05 to 300 ppm by mass. This tends to allow the effects of the present invention to be obtained more favorably. When the total content of the alkali metal is 0.05 ppm by mass or more, more satisfactory reactivity, hydrolysis resistance, tensile strength, and tensile elongation are obtained, and when the total content is 500 ppm by mass or less, the reactivity tends to be further improved at the time of synthesizing the polymer.

The content of a glycerin-derived structural unit in 100% by mass of the polymer of the present invention is preferably 1500 ppm by mass or less, more preferably 1000 ppm by mass or less, even more preferably 500 ppm by mass or less, and particularly preferably 0 ppm by mass (not included).

This tends to allow the effects to be obtained more favorably.

The polymer may be modified. The modification is not particularly limited, and examples include the modifications described in connection with the 1,6-hexanediol derivative. Among them, modification with (meth)acrylic acid is preferred. Particularly, in a case where the polymer is a polyester, it is preferable that the polymer is subjected to modification with (meth)acrylic acid.

The number average molecular weight (Mn) of the polymer of the present invention is preferably 500 to 1,000,000, and more preferably 3,000 to 500,000. **In** the present specification, the number average molecular weight (Mn) of the polymer is a value measured by gel permeation chromatography (GPC).

**In** the following description, the details of a polyester and a polyurethane among the above-described polymers will be described; however, the polymer is not limited to a polyester and a polyurethane.

### <<Polyester>>

The polyester of the present invention is obtained by reacting the 1,6-hexanediol composition of the present invention. Specifically, the polyester of the present invention is a polycondensation product synthesized by subjecting a carboxylic acid and an alcohol to dehydration condensation to form ester bonds, and at least the 1,6-hexanediol composition of the present invention is used as the alcohol. Therefore, the polyester of the present invention is a reaction product (polycondensation product) obtained by a polycondensation reaction of a carboxylic acid and an alcohol, and the polyester of the present invention has at least a structural unit derived from the 1,6-hexanediol composition of the present invention, and also optionally contains a structural unit derived from the glycerin included in the 1,6-hexanediol composition.

The polyester of the present invention may be a polyester polyol.

Examples of the polyester polyol include a condensation-based polyester polyol and a lactone-based polyester polyol. The condensation-based polyester polyol is a reaction product of a low molecular weight polyhydric alcohol (a low molecular weight polyol such as ethylene glycol (EG), diethylene glycol, propylene glycol (PG), dipropylene glycol, (1,3- or 1,4-) butanediol, pentanediol, neopentyl glycol, hexanediol, cyclohexanedimethanol, glycerin, 1,1,1-trimethylolpropane (TMP), 1,2,5-hexanetriol, pentaerythritol, or 1,4-cyclohexanedimethanol; a sugar such as sorbitol; or the like) and a polyvalent basic carboxylic acid (glutaric acid, adipic acid, azelaic acid, fumaric acid, maleic acid, pimelic acid, suberic acid, sebacic acid, phthalic acid, terephthalic acid, isophthalic acid, dimer acid, pyromellitic acid, oligomer acid, hexahydrophthalic anhydride, 1,4-cyclohexanedicarboxylic acid, or the like). The lactone-based polyester polyol is, for example, a polycaprolactone polyol obtained by subjecting a lactone such as ε-caprolactone, α-methyl-ε-caprolactone, or ε-methyl-ε-caprolactone to ring-opening polymerization.

Examples of the carboxylic acid include aliphatic polyvalent carboxylic acids such as succinic acid, adipic acid, azelaic acid, sebacic acid, glutaric acid, pimelic acid, suberic acid, dodecanedicarboxylic acid, maleic acid, and fumaric acid; alicyclic polyvalent carboxylic acids such as 1,3-cyclopentanedicarboxylic acid, 1,2-cyclohexanedicarboxylic acid, 1,3-cyclohexanedicarboxylic acid, 1,4-cyclohexanedicarboxylic acid, hexahydrophthalic anhydride, and 1,4-cyclohexanedicarboxylic acid; aromatic polyvalent carboxylic acids such as orthophthalic acid, isophthalic acid, terephthalic acid, naphthalenedicarboxylic acid, biphenyldicarboxylic acid, 1,2-bis(phenoxy)ethane-p,p'-dicarboxylic acid, and trimellitic acid; aliphatic monocarboxylic acids such as acetic acid, propionic acid, 2-ethylhexanoic acid, acrylic acid, and methacrylic acid; aromatic monocarboxylic acids such as benzoic acid, p-tertiary butylbenzoic acid, and p-hydroxybenzoic acid; various fatty acids derived from animal and vegetable oils, such as soybean oil fatty acids, tall oil fatty acids, linoleic acid, and eicosapentaenoic acid; and anhydrides thereof. These may be used singly, or two or more kinds thereof may be used in combination. Among them, aliphatic carboxylic acids are preferred, and adipic acid is more preferred.

The alcohol that can be used in addition to the 1,6-hexanediol composition of the present invention is not particularly limited as long as it is a compound having a hydroxyl group (an alcoholic hydroxyl group or a phenolic hydroxyl group). Examples of the alcohol include aliphatic monoalcohols such as ethanol, butanol, and 2-ethylhexanol; aliphatic polyols such as ethylene glycol, neopentyl glycol, and trimethylolpropane; aromatic monohydric/polyhydric phenols such as phenol, cresol, and bisphenolA; and ethylene oxide extension products and hydrogenated alicyclic alcohols thereof. These may be used singly, or two or more kinds thereof may be used in combination.

The content of the 1,6-hexanediol composition of the present invention in 100% by mass of the alcohol, which is a reaction raw material of the polyester, is preferably 10% to 100% by mass, and more preferably 50% to 100% by mass. This tends to allow the effects to be obtained more favorably.

The polyester (polyester polyol in some cases) of the present invention can be obtained by a known production method for polyesters. Specifically, the polyester can be synthesized by a production method of reacting the above-described carboxylic acid with the above-described alcohol at a reaction temperature of 150°C to 280°C while removing any produced water from the system. Furthermore, a reaction catalyst, an oxidation inhibitor, and the like may be used in combination during the synthesis.

The content of a structural unit derived from the 1,6-hexanediol composition of the present invention in 100% by mass of the polyester of the present invention is preferably 10% to 70% by mass, and more preferably 20% to 70% by mass. This tends to allow the effects to be obtained more favorably.

In the present specification, the content of each structural unit in the polyester is measured by NMR.

The total content of a structural unit derived from (A) the compound having two or more secondary hydroxyl groups in one molecule in 100% by mass of the polyester of the present invention is preferably 70 ppm by mass or less, more preferably 35 ppm by mass or less, even more preferably 7 ppm by mass or less, and particularly preferably 0 ppm by mass (not included).

This tends to allow the effects to be obtained more favorably.

The total content of a structural unit derived from (B) 6-hydroxyhexanal and/or a derivate thereof in 100% by mass of the polyester of the present invention is preferably 700 ppm by mass or less, more preferably 350 ppm by mass or less, even more preferably 140 ppm by mass or less, and particularly preferably 0 ppm by mass (not included).

This tends to allow the effects to be obtained more favorably.

In the polyester of the present invention, the total content of the alkali metal elements (preferably, the total content of either or both of sodium metal element and potassium metal element) is preferably 0.05 to 500 ppm by mass, more preferably 0.05 to 400 ppm by mass, and even more preferably 0.05 to 300 ppm by mass. When the total content of the alkali metal is 0.05 ppm by mass or more, more satisfactory reactivity, hydrolysis resistance, tensile strength, and tensile elongation are obtained, and when the total content is 500 ppm by mass or less, the reactivity tends to be further improved at the time of synthesizing urethane using the polyester of the present invention as a raw material.

The content of a glycerin-derived structural unit in 100% by mass of the polyester of the present invention is preferably 1400 ppm by mass or less, more preferably 1050 ppm by mass or less, even more preferably 700 ppm by mass or less, and particularly preferably 0 ppm by mass (not included).

This tends to allow the effects to be obtained more favorably.

The number average molecular weight (Mn) of the polyester of the present invention is preferably 500 to 120,000, and more preferably 3,000 to 50,000. Incidentally, in the present specification, the number average molecular weight (Mn) of the polyester is a value measured by gel permeation chromatography (GPC).

### <<Polyurethane>>

The polyurethane of the present invention is a reaction product obtained by reacting a polyol produced using the 1,6-hexanediol composition of the present invention as a reaction raw material, with a polyisocyanate. Furthermore, if necessary, a polyol produced without using a 1,6-hexanediol composition as a reaction raw material, a chain extending agent, a chain terminating agent, a crosslinking agent, and the like may be used in combination as reaction raw materials for the polyurethane of the present invention.

Therefore, the polyurethane of the present invention has a polyol-derived structural unit and a polyisocyanate-derived structural unit, and the polyurethane has at least a structural unit derived from the 1,6-hexanediol composition of the present invention and also optionally contains a structural unit derived from the glycerin included in the 1,6-hexanediol composition of the present invention.

Examples of the polyol include polyols such as a polycarbonate polyol, a polyether polyol, and a polyester polyol. These polyols may or may not be produced using the 1,6-hexanediol composition as a reaction raw material, and it is desirable that the polyurethane of the present invention uses, at least as a reaction raw material, a polyol produced using the 1,6-hexanediol composition as a reaction raw material.

A polycarbonate polyol is a polyol obtained by a reaction between a glycol and a carbonate. Examples of the glycol include various saturated or unsaturated glycols such as the 1,6-hexanediol composition of the present invention, diethylene glycol, ethylene glycol, triethylene glycol, 1,2-propanediol, 1,3-propanediol, 1,3-butanediol, 1,4-butanediol, neopentyl glycol, pentanediol, 3-methyl-1,5-pentanediol, octanediol, 1,4-butanediol, dipropylene glycol, tripropylene glycol, polytetramethylene ether glycol, 2-methyl-1,3-propanediol, and 2-ethyl-2-butyl-1,3-propanediol; and alicyclic glycols such as 1,4-cyclohexane diglycol and 1,4-cyclohexane dimethanol.

Furthermore, examples of the carbonate include dialkyl carbonates (dimethyl carbonate, diethyl carbonate, and the like), ethylene carbonate, and diphenyl carbonate.

The polyether polyol is, for example, a polyether polyol obtained by subjecting an alkylene oxide to addition polymerization using various glycols as an initiator. Examples of the alkylene oxide include ethylene oxide, propylene oxide, butylene oxide, and tetrahydrofuran. Examples of the various glycols include glycols similar to the above-described glycol of polycarbonate diol.

As the polyol, in addition to those described above, polycaprolactone polyol, polyacrylic polyol, dimer diol, polybutadiene polyol, hydrogenated polybutadiene polyol, and the like can be used. These polyols may be used singly, or two or more kinds thereof may be used in combination.

As the chain extending agent, for example, aliphatic polyol compounds such as ethylene glycol, diethylene glycol, triethylene glycol, 1,2-propanediol, 1,3-propanediol, 1,3-butanediol, 1,4-butanediol, hexamethylene glycol, saccharose, methylene glycol, glycerin, sorbitol, and neopentyl glycol; aromatic polyol compounds such as bisphenol A, 4,4'-dihydroxydiphenyl, 4,4'-dihydroxydiphenyl ether, 4,4'-dihydroxydiphenyl sulfone, hydrogenated bisphenol A, and hydroquinone; water; and amine compounds such as ethylenediamine, 1,2-propanediamine, 1,6-hexamethylenediamine, piperazine, 2-methylpiperazine, 2,5-dimethylpiperazine, isophorone diamine, 4,4'-dicyclohexylmethanediamine, 3,3'-dimethyl-4,4'-dicyclohexylmethanediamine, 1,2-cyclohexanediamine, 1,4-cyclohexanediamine, aminoethylethanolamine, hydrazine, diethylenetriamine, triethylenetetramine, isophorone diamine, and 4,4'-methylenebis(2-chloroaniline), can be used. Regarding these chain extending agents, those derived from biomass resources can also be used. These chain extending agents may be used singly, or two or more kinds thereof may be used in combination. Among them, neopentyl glycol, 1,4-butanediol, trimethylolpropane, isophorone diamine, and 4,4'-methylenebis(2-chloroaniline) are more preferred.

As the polyol, it is particularly preferable to use a polyester polyol produced using the composition of the present invention as a raw material, and the content of the polyester polyol produced using the composition of the present invention as a raw material in 100% by mass of the polyol is preferably 10% to 100% by mass, and more preferably 50% to 100% by mass.

As the polyisocyanate, for example, aromatic polyisocyanates such as 1,3- and 1,4-phenylene diisocyanate, 1-methyl-2,4-phenylene diisocyanate, 1-methyl-2,6-phenylene diisocyanate, 1-methyl-2,5-phenylene diisocyanate, 1-methyl-2,6-phenylene diisocyanate, 1-methyl-3,5-phenylene diisocyanate, 1-ethyl-2,4-phenylene diisocyanate, 1-isopropyl-2,4-phenylene diisocyanate, 1,3-dimethyl-2,4-phenylene diisocyanate, 1,3-dimethyl-4,6-phenylene diisocyanate, 1,4-dimethyl-2,5-phenylene diisocyanate, diethylbenzene diisocyanate, diisopropylbenzene diisocyanate, 1-methyl-3,5-diethylbenzene diisocyanate, 3-methyl-1,5-diethylbenzene-2,4-diisocyanate, 1,3,5-triethylbenzene-2,4-diisocyanate, naphthalene-1,4-diisocyanate, naphthalene-1,5-diisocyanate, 1-methyl-naphthalene-1,5-diisocyanate, naphthalene-2,6-diisocyanate, naphthalene-2,7-diisocyanate, 1,1-dinaphthyl-2,2'-diisocyanate, biphenyl-2,4'-diisocyanate, biphenyl-4,4'-diisocyanate, 3,3'-dimethylbiphenyl-4,4'-diisocyanate, 4,4'-diphenylmethane diisocyanate, 2,2'-diphenylmethane diisocyanate, diphenylmethane-2,4-diisocyanate, and toluene diisocyanate; aliphatic polyisocyanates such as tetramethylene diisocyanate, 1,6-hexamethylene diisocyanate, dodecamethylene diisocyanate, and trimethylhexamethylene diisocyanate; alicyclic polyisocyanates such as 1,3-cyclopentylene diisocyanate, 1,3-cyclohexylene diisocyanate, 1,4-cyclohexylene diisocyanate, 1,3-di(isocyanatomethyl)cyclohexane, 1,4-di(isocyanatomethyl)cyclohexane, lysine diisocyanate, isophorone diisocyanate, 4,4'-dicyclohexylmethane diisocyanate, 2,4'-dicyclohexylmethane diisocyanate, 2,2'-dicyclohexylmethane diisocyanate, and 3,3'-dimethyl-4,4'-dicyclohexylmethane diisocyanate; and the like can be used. Regarding these polyisocyanates, those derived from biomass resources can also be used. These may be used singly, or two or more kinds thereof may be used in combination. Among them, aromatic polyisocyanates are preferred, and 4,4'-diphenylmethane diisocyanate and toluene diisocyanate are more preferred.

For the purpose of controlling the molecular weight of the obtained polyurethane, chain terminating agents having one active hydrogen group may be used as necessary. Examples of these chain terminating agents include aliphatic monohydroxy compounds having a hydroxyl group, such as methanol, ethanol, propanol, butanol, and hexanol; and aliphatic monoamines having an amino group, such as morpholine, diethylamine, dibutylamine, monoethanolamine, and diethanolamine. These may be used singly, or two or more kinds thereof may be used as a mixture.

For the purpose of increasing the heat resistance or strength of the obtained polyurethane, crosslinking agents having three or more active hydrogen groups or isocyanate groups can be used as necessary.

The polyurethane of the present invention can be obtained by a known production method for polyurethanes. Specifically, for example, a method for producing a polyurethane by preparing the above-mentioned polyol, the above-mentioned polyisocyanate, and the above-mentioned chain extending agent and allowing them to react, may be mentioned. It is preferable that such a reaction is carried out at, for example, a temperature of 50°C to 100°C for 3 to 10 hours. Furthermore, the reaction may be carried out in an organic solvent.

As the organic solvent, for example, ketone solvents such as N,N-dimethylformamide, N,N-dimethylacetamide, N-methyl-2-pyrrolidone, methyl ethyl ketone, methyl-n-propyl ketone, acetone, and methyl isobutyl ketone; ester solvents such as methyl formate, ethyl formate, propyl formate, methyl acetate, ethyl acetate, isopropyl acetate, n-butyl acetate, isobutyl acetate, and secondary butyl acetate; and alcohol solvents such as methanol, ethanol, isopropyl alcohol, and butanol, can be used. Furthermore, regarding these organic solvents, those induced from biomass resources may also be used. These organic solvents may be used singly, or two or more kinds thereof may be used in combination.

When the polyurethane is produced using, as a raw material, a polyester polyol produced using the 1,6-hexanediol composition of the present invention as a raw material, the content of the structural unit derived from the polyester polyol of the present invention in 100% by mass of the polyurethane is preferably 10% to 90% by mass, and more preferably 20% to 90% by mass. This tends to allow the effects to be obtained more favorably.

Furthermore, the content of a structural unit derived from the 1,6-hexanediol composition of the present invention in 100% by mass of the polyurethane of the present invention is preferably 1% to 63% by mass, and more preferably 2% to 63% by mass. This tends to allow the effects to be obtained more favorably.

In the present specification, the content of each structural unit in the polyurethane is measured by NMR.

The total content of the structural unit derived from (A) the compound having two or more secondary hydroxyl groups in one molecule in 100% by mass of the polyurethane of the present invention is preferably 63 ppm by mass or less, more preferably 32 ppm by mass or less, even more preferably 6 ppm by mass or less, and particularly preferably 0 ppm by mass (not included).

This tends to allow the effects to be obtained more favorably.

The total content of the structural unit derived from (B) 6-hydroxyhexanal and/or a derivative thereof in 100% by mass of the polyurethane of the present invention is preferably 945 ppm by mass or less, more preferably 630 ppm by mass or less, even more preferably 315 ppm by mass or less, and particularly preferably 0 ppm by mass (not included). This tends to allow the effects to be obtained more favorably.

In the polyurethane of the present invention, the total content of the alkali metal elements (preferably, the total content of either or both of sodium metal element and potassium metal element) is preferably 0.05 to 500 ppm by mass, more preferably 0.05 to 400 ppm by mass, and even more preferably 0.05 to 300 ppm by mass. When the total content of the alkali metal is 0.05 ppm by mass or more, more satisfactory reactivity, hydrolysis resistance, tensile strength, and tensile elongation tend to be obtained.

The content of a glycerin-derived structural unit in 100% by mass of the polyurethane of the present invention is preferably 1260 ppm by mass or less, more preferably 945 ppm by mass or less, even more preferably 630 ppm by mass or less, and particularly preferably 0 ppm by mass (not included).

This tends to allow more favorable tensile strength and tensile elongation to be obtained.

The number average molecular weight (Mn) of the polyurethane of the present invention is preferably 5,000 to 1,000,000, and more preferably 10,000 to 500,000. This tends to allow the effects to be obtained more favorably. Incidentally, in the present specification, the number average molecular weight (Mn) of the polyurethane is a value measured by gel permeation chromatography (GPC).

The polymer of the present invention can be used for various use applications. Specifically, the polymer can be used for a variety of use applications such as artificial leather, synthetic leather, shoes, thermoplastic resins, foamed resins, thermosetting resins, paints, laminating adhesives, elastic fibers, urethane raw materials, automobile parts, sporting goods, vibration insulating materials, vibration damping materials, fiber treatment agents, and binders.

The polyester of the present invention can be used for various use applications. Specifically, the polyester can be used for a variety of use applications such as solvent-based, water-based, and powdered coating materials for metals, automobiles, woodworking, and plastics; modifying agents for packaging materials and containers, optical films, and molded articles; surface layers, intermediate layers, foamed layers, and adhesive layers of artificial leather and synthetic leather; adhesives such as laminating adhesives; shoes, thermoplastic resins, foamed resins, thermosetting resins, elastic fibers, polyurethane raw materials, automobile parts, and sporting goods.

The polyurethane of the present invention can be used for various use applications. Specifically, the polyurethane can be used for a variety of use applications such as surface layers, intermediate layers, foamed layers, and adhesive layers of artificial leather and synthetic leather; various coating agents such as paints, metal surface treatment agents, and film primers; binders for inkjet printing, inks, textile printing, and glass fiber bundling agents; shoes, thermoplastic resins, foamed resins, thermosetting resins, adhesives such as laminating adhesives, vibration insulating materials, vibration damping materials, automobile parts, sporting goods, and fiber treatment agents.

An epoxy-based polymer produced using the epoxy group-containing 1,6-hexanediol derivative as a raw material can be used for various use applications. Specifically, the epoxy-based polymer can be used for a variety of use applications such as various coating agents such as paints and metal surface treatment agents; and molded products of matrix resins and the like for wind turbines.

An acrylic polymer produced using the (meth)acryloyl group-containing 1,6-hexanediol derivative as a raw material can be used for various use applications. Specifically, the acrylic polymer can be used for a variety of use applications such as various coating agents such as paints, metal surface treatment agents, and film coatings; and binders for inks and UV inkjet printing.

Furthermore, the 1,6-hexanediol composition of the present invention can also be used for the modification of PBS resins, PET resins, PTT resins, PBT resins, and the like by adding the composition.

In the present specification, the notation "to" means values equal to or greater than the value before the notation "to", and equal to or less than the value after the notation "to". Furthermore, in the present specification, when a plurality of numerical value ranges are disclosed by the notation "to" for a certain characteristic or the like, the upper limit and the lower limit of each numerical value range can be used in any combination. For example, when two numerical value ranges of 0.001 to 500 ppm by mass and 0.05 to 250 ppm by mass are disclosed for the content of a certain compound, it is implied that numerical value ranges of 0.001 to 250 ppm by mass and 0.05 to 500 ppm by mass are also disclosed in addition to the ranges of 0.001 to 500 ppm by mass and 0.05 to 250 ppm by mass.

### EXAMPLES

Hereinafter, the present invention will be described specifically by way of Examples and Comparative Examples; however, the present invention is not intended to be limited to these only.

### (Production Example 1) (Preparation of genetically modified Escherichia coli)

A plasmid A was prepared, in which glycerol dehydratase α, β, and γ subunit genes derived from Citrobacter freundii, and a dehydratase reactivation factor gene were introduced between BamHI and HindIII restriction enzyme cleavage sites of pACYC184 (NIPPON GENE CO., LTD.).

A plasmid B was prepared, in which Escherichia coli-derived HpaI gene as 4,6-dihydroxy-2-oxo-hexanoate aldolase, Escherichia coli-derived HpcG gene as 4,6-dihydroxy-2-oxo-hexanoate 4-dehydratase, Arabidopsis thaliana-derived NADPH-dependent oxidoreductase 2-alkenal reductase (GenBank: CAC01710.1) gene as 6-hydroxy-3,4-dehydro-2-oxohexanoate 3-reductase, PanE gene of Lactococcus lactis as 6-hydroxy-2-oxohexanoate 2-reductase, and Clostridium difficile-derived HadA gene as 2,6-dihydroxy-hexanoate CoA-transferase and 6-hydroxyhexanoyl-CoA-transferase were introduced between BamHI and EcoRI restriction enzyme cleavage sites of the multiple cloning site (MCS) of pUC19 (NIPPON GENE CO., LTD.).

A plasmid C was prepared, in which Clostridium difficile-derived HadB and HadC genes as α and β subunit genes of 2-hydroxyisocaproyl-CoA dehydratase, Clostridium difficile-derived HadI gene as 2-hydroxyisocaproyl-CoA dehydratase activating enzyme, Treponema denticola-derived trans-2-enoyl-CoA reductase (GenBank: AE017248) gene as 6-hydroxy-2,3-dehydro-hexanoyl-CoA 2,3-reductase, Nocardia iowensis-derived carboxylic acid reductase (GenBank: AAR91681.1) gene as 6-hydroxyhexanoate 1-reductase, and Rhodococcus-derived 6-hydroxyhexanoate dehydrogenase (GenBank: AAN37489.1) gene as 6-hydroxyhexanal 1-reductase were introduced into pCOLADuet-1 (Novagen, Inc.) such that HadB, HadC, and HadI genes were introduced into MCS1 and the other genes were introduced into MCS2.

In the present section, the term gene refers to an open reading frame containing a stop codon encoding each enzyme, and each gene is introduced into a plasmid such that a sequence containing a T7 promoter and a ribosome binding site, is present upstream of the gene, and a sequence containing a T7 terminator is present downstream of the gene. Each gene can be expressed in a large amount in a host strain of Escherichia coli, in which T7 RNA polymerase is expressed by appropriate induction of expression.

Plasmids A, B, and C were sequentially transformed in chemically competent cells of BL21 Star (DE3) (Invitrogen Corporation) by a heat shock method, and the transformants were selected on an LB plate containing appropriate antibiotics. As a result, a BL21 Star (DE3) strain containing all the plasmids A, B, and C was obtained. In this way, an Escherichia coli having genes encoding ten enzymes of the 1,6-hexanediol pathway consisting of 4,6-dihydroxy-2-oxo-hexanoate aldolase (2A in the drawings of JP 6680671 B2), 4,6-dihydroxy-2-oxo-hexanoate 4-dehydratase (2B in the drawings of JP 6680671 B2), 6-hydroxy-3,4-dehydro-2-oxohexanoate 3-reductase (2C in the drawings of JP 6680671 B2), 6-hydroxy-2-oxohexanoate 2-reductase (2D in the drawings of JP 6680671 B2), 2,6-dihydroxy-hexanoate CoA-transferase (2E in the drawings of JP 6680671 B2), 2,6-dihydroxy-hexanoyl-CoA 2-dehydratase (2F in the drawings of JP 6680671 B2), 6-hydroxy-2,3-dehydrohexanoyl-CoA 2,3-reductase (2G in the drawings of JP 6680671 B2), 6-hydroxyhexanoyl-CoA-transferase (4F3 in the drawings of JP 6680671 B2), 6-hydroxyhexanoate 1-reductase (5R in the drawings of JP 6680671 B2), and 6-hydroxyhexanal 1-reductase (5S in the drawings of JP 6680671 B2), was prepared.

### (Example 1: Preparation of 1,6-hexanediol composition 1 (1,6-HD composition 1))

The Escherichia coli was inoculated into an autoclave-sterilized medium (carbon sources: glucose, glycerin; nitrogen source: enzyme extract; inorganic salts: potassium phosphate, potassium hydroxide, vitamin B12; antibiotics: carbenicillin, kanamycin, chloramphenicol; pH: 7.0, among the above-described components, glucose and glycerin were raw materials derived from biomass resources), and cultured at 30°C for 2 to 3 hours under aerobic conditions. Thereafter, when the optical density at 600 nm of the Escherichia coli reached 0.3 to 0.6, isopropyl-β-thiogalactopyranoside was added to a final concentration of 0.5 mM, iron(II) sulfate was added to a final concentration of 10 µM, culturing was further carried out at 30°C for 3 hours, and the enzymes of the 1,6-HD pathway were expressed. After the expression, an appropriate amount of carbon sources (glucose and glycerin) were added thereto, and the inside of the culture vessel was placed under a nitrogen atmosphere to create anaerobic conditions. Under these conditions, culture was carried out at 30°C for 48 hours, and 1,6-hexanediol was produced (step (1)). The culture fluid was centrifuged at 4°C for 20 minutes, the supernatant was collected and filtered using an appropriate membrane filter having a pore size of 0.2 to 0.4 µm, and a 1,6-hexanediol composition was obtained as a filtrate.

### [Purification of 1,6-hexanediol composition]

### <Step (a): Ion exchange to remove cations>

The cations included in the 1,6-hexanediol composition were removed. In step (a), cation exchange was carried out in a batch manner. The temperature for bringing into contact with a cation exchange resin was set to 40°C, DIAION SK1BH manufactured by Mitsubishi Chemical Corporation was added as the cation exchange resin into the 1,6-hexanediol composition, followed by stirring for 3 hours. After the stirring, filtration was performed, and a 1,6-hexanediol composition A was obtained as a filtrate.

### <Step (b): Ion exchange to remove anions>

The anions included in the 1,6-hexanediol composition A were removed. In step (b), anion exchange was carried out in a batch manner. The temperature for bringing into contact with an anion exchange resin was set to 40°C, DIAION SA10AOH manufactured by Mitsubishi Chemical Corporation was added as the anion exchange resin into the 1,6-hexanediol composition, followed by stirring for 3 hours. After the stirring, filtration was performed, and a 1,6-hexanediol composition B was obtained as a filtrate.

### <Step (c): Step of removing water>

Moisture included in the 1,6-hexanediol composition was removed. A thin film type distiller was used as the apparatus for step (c). The jacket temperature was set to 70°C, the 1,6-hexanediol-containing composition was continuously introduced, and water was distilled out from the top part. Simultaneously with the distillation of water, dehydrated 1,6-hexanediol composition C was continuously withdrawn from the bottom part as a bottom product. The moisture concentration in this 1,6-hexanediol composition C was 0.020% by mass (200 ppm by mass).

### <Step (d): Distillation separation of low boiling point components>

Components included in the 1,6-hexanediol composition C and having a boiling point lower than that of 1,6-hexanediol were removed in a continuous distillation column. As the distillation column in step (d), an Oldershaw distillation column was used. The 1,6-hexanediol composition C obtained in step (c) was continuously fed into the distillation column, and the column top temperature was controlled at a constant temperature of 240°C. Continuous distillation was carried out from the top part of the column, continuous withdrawal was carried out from the bottom of the column, removal of low boiling point components in the 1,6-hexanediol composition C was carried out, and a 1,6-hexanediol composition D from which components having a lower boiling point than that of 1,6-hexanediol had been removed was withdrawn from the bottom of the column.

### <Step (e): Distillation separation of high boiling point components>

Components included in the 1,6-hexanediol composition D and having a boiling point higher than that of 1,6-hexanediol were removed in a continuous distillation column. As the distillation column in step (e), an Oldershaw distillation column was used. The 1,6-hexanediol composition D obtained in step (d) was continuously fed into the distillation column, and the column bottom temperature was controlled to be constant at 260°C. Continuous withdrawal was carried out from the bottom of the column, and removal of high boiling point components in the 1,6-hexanediol composition D was carried out. A 1,6-hexanediol composition E from which components having a higher boiling point than that of 1,6-hexanediol had been removed (1,6-HD composition 1) was obtained from the top of the column (column top distillate).

The analysis results for the obtained 1,6-HD composition 1 are shown below. Incidentally, in the analysis of the 1,6-HD composition, the detection limits for glycerin, glucose, 1,4-cyclohexanediol, 6-hydroxyhexanal, potassium metal, and sodium metal element were 5.0 ppm by mass, 5.0 ppm by mass, 0.3 ppm by mass, 10.0 ppm by mass, 3.0 ppb by mass, and 3.0 ppb by mass, respectively. Furthermore, quantification of potassium metal element and sodium metal element was performed by ICP-MS analysis, and the analysis of the others was performed by HPLC analysis and GC analysis.
Glycerin: 800 ppm by mass
Glucose: Detection limit or less
1,4-Cyclohexanediol: Detection limit or less
6-Hydroxyhexanal: 500 ppm by mass
Potassium metal element: 2 ppm by mass
Sodium metal element: 2 ppm by mass

### Details of HPLC analysis

· Apparatus: HPLC apparatus manufactured by SHIMADZU CORPORATION (system controller: SCL-10A, liquid delivery unit: LC-10AD, column oven: CTO-10A, autosampler: SIL-10AD, low pressure gradient unit: FCV-10AL, online degasser: DGU-14A)
· Column: TSKgel OApak-A
· Guard column: TSKgel OAPak-P
· Column temperature: 40°C
· Eluent (isocratic): 0.75 mM sulfuric acid
· Flow rate: 0.8 mL/min
· Injection volume: 10.0 µL
· Detector: RI (RID-10A)

### Details of GC analysis

· Apparatus: GC-MS manufactured by Agilent Technologies, Inc. (GC7890B/MSD5977B) · Column: Agilent J&W GC Column-DB-1
· Carrier gas: He
· Flow rate: 0.966 mL/min
· Linear velocity: 99.5 cm/sec
· Injection volume: 1 µL
· Split ratio: 50
· Column temperature: 75°C (hold: 2 min) - 10°C/min (temperature increase rate, 22.5 min) - 300°C (Hold: 5.5 min)
· Detector: FID

### (Synthesis of polyester resin)

546.0 g (4.62 mol) of the 1,6-HD composition 1 and 602.3 g (4.12 mol) of adipic acid were introduced into a reaction vessel equipped with a stirring rod, a temperature sensor, and a rectification tube, 0.05 g of tetraisopropyl titanate was added thereto as a catalyst, and then while allowing dry nitrogen to flow into the flask and stirring the contents, a dehydration condensation reaction was carried out by heating to 210°C to 230°C. The reaction was continued until the acid value reached 0.2, and a polyester polyol having a hydroxyl group value of 55.9 and a number average molecular weight of 2000 was obtained.

Evaluation of the obtained polyester was carried out by the following methods, and the analysis results are shown below.

### Reaction time: Reaction time until the acid value reached 0.2 (rating in three grades)

Shorter than 6 hours: A
6 hours or longer and shorter than 8 hours: B
Longer than 8 hours: C

Number of colors: The number of colors was evaluated using the Gardner Color (JIS K0071; 1998) to a time point at which the polyester resin that had melted at 100°C solidified (a smaller number indicates less coloring)

Odor: 1000 g of the polyester polyol was placed in a 1-kg metal can and was left to stand in a dryer at 100°C for 3 hours to completely melt, and then the intensity of odor at the time of opening the lid was subjected to sensory evaluation: "A: Almost odorless", "B: Slight odor", "C: Strong odor", "D: Quite strong odor"

### (Evaluation results)

Reaction time: A Number of colors: 1 Odor: C

### (Synthesis of polyurethane resin)

400 g (0.200 mol) of the above-described polyester polyol and 40.0 g (0.384 mol) of neopentyl glycol were dissolved in 542 g of DMF solvent in a four-necked flask equipped with a thermometer, a stirrer, and an inert gas inlet port. Next, 101.7 g (0.584 mol) of tolylene diisocyanate and 101.7 g (0.584 mol) of CORONATE T-80 were added thereto at 40°C, the mixture was allowed to react at 90°C until the NCO% reached 0.1% or less, and a urethane solution with a non-volatile content of 50% was obtained.

Evaluation of the obtained polyurethane was carried out by the following methods, and the analysis results are shown below.

Reaction time: Time taken from the charging of TDI until the NCO% reached 0.1% or less
Shorter than 6 hours: A
6 hours or longer and shorter than 8 hours: B
8 hours or longer and shorter than 10 hours: C
10 hours or longer: D

Film physical properties: After blending the obtained urethane resin with a polyisocyanate crosslinking agent (solid content of urethane resin/solid content of crosslinking agent = 10/1 in mass ratio), immediately the mixture was preliminarily dried in a Werner Mathis at 70°C for 1 minute, and then drying was carried out at 120°C for 2 minutes to obtain a urethane resin coating film. This coating film was aged at 40°C for 2 days, and then the film physical properties (initial; breaking strength and breaking elongation) were measured using an AUTOGRAPH (manufactured by SHIMADZU CORPORATION) in an environment at 23°C and 65%RH. Furthermore, as a hydrolysis resistance test, the same urethane coating film was left to stand for one week under the conditions of 70°C and 95%RH, moisture was further dried at normal temperature for one day, subsequently the film physical properties were measured in the same manner as for the initial state, and the degree of deterioration of the coating film was measured.

Breaking strength (initial): "A: 50 MPa or more", "B: 40 or more and less than 50 MPa", "C: 30 or more and less than 40 MPa", "D: Less than 30 MPa"

Breaking elongation (initial): "A: 500% or more", "B: 400% or more and less than 500%", "C: 300% or more and less than 400%", "D: Less than 300%"

Hydrolysis resistance (evaluated based on the retention ratio of the breaking strength from the initial value and the breaking elongation after a hydrolysis resistance test):
"A: Retention ratio of breaking strength 80% or more, and breaking elongation 100% or more"
"B: Retention ratio of breaking strength 70% or more and less than 80%, and breaking elongation 100% or more"
"C: Retention ratio of breaking strength 50% or more and less than 70%, and breaking elongation 100% or more"
"D: Retention ratio of breaking strength less than 50%, or breaking elongation less than 100%"

### (Evaluation results)

Reaction time: B Breaking strength of coating film: B Breaking elongation of coating film: C Hydrolysis resistance: A

### (Example 2: Preparation of 1,6-hexanediol composition 2 (1,6-HD composition 2))

Purification of the 1,6-hexanediol composition obtained by the bio method in Example 1 was repeated, and a 1,6-hexanediol composition 2 having a glycerin content of 150 ppm by mass and a 6-hydroxyhexanal content of 100 ppm by mass was obtained.

The analysis results of the obtained 1,6-HD composition 2 are shown below.
Glycerin: 150 ppm by mass
Glucose: Detection limit or less
1,4-Cyclohexanediol: Detection limit or less
6-Hydroxyhexanal: 100 ppm by mass
Potassium metal element: 1 ppm by mass
Sodium metal element: 1 ppm by mass

The synthesis and evaluation of the polyester resin, and the synthesis and evaluation of the polyurethane resin were carried out in the same manner as in Example 1.

### (Evaluation results)

Evaluation of polyester Reaction time: A Number of colors: < 1 Odor: B Evaluation of polyurethane Reaction time: B Breaking strength of coating film: B Breaking elongation of coating film: B Hydrolysis resistance: B

### (Example 3: Preparation of 1,6-hexanediol composition 3 (1,6-HD composition 3))

Purification of the 1,6-hexanediol composition obtained by the bio method in Example 1 was repeated, and a 1,6-hexanediol composition 3 having a glycerin content of 5 ppm by mass and a 6-hydroxyhexanal content of 10 ppm by mass was obtained.

The analysis results of the obtained 1,6-HD composition 3 are shown below.
Glycerin: 5 ppm by mass
Glucose: Detection limit or less
1,4-Cyclohexanediol: Detection limit or less
6-Hydroxyhexanal: 10 ppm by mass
Potassium metal element: Detection limit or less
Sodium metal element: Detection limit or less

The synthesis and evaluation of the polyester resin, and the synthesis and evaluation of the polyurethane resin were carried out in the same manner as in Example 1.

### (Evaluation results)

Evaluation of polyester Reaction time: A Number of colors: < 1 Odor: B Evaluation of polyurethane Reaction time: B Breaking strength of coating film: A Breaking elongation of coating film: A Hydrolysis resistance: B

### (Example 4: Preparation of 1,6-hexanediol composition 4 (1,6-HD composition 4))

A 1,6-hexanediol composition 4 (1,6-HD composition 4) was prepared by adding potassium acetate to the 1,6-HD composition 3 obtained in Example 3 such that the potassium metal element concentration was 300 ppm by mass, and adding sodium acetate to the 1,6-HD composition 3 such that the sodium metal element concentration was 200 ppm by mass.

The synthesis and evaluation of the polyester resin, and the synthesis and evaluation of the polyurethane resin were carried out in the same manner as in Example 1.

### (Evaluation results)

Evaluation of polyester Reaction time: A Number of colors: < 1 Odor: B Evaluation of polyurethane Reaction time: A Breaking strength of coating film: A Breaking elongation of coating film: A Hydrolysis resistance: A

### (Example 5: Preparation of 1,6-hexanediol composition 5 (1,6-HD composition 5))

A 1,6-hexanediol composition 5 (1,6-HD composition 5) was prepared by adding glycerin to the 1,6-HD composition 3 obtained in Example 3 such that the glycerin content was 150 ppm by mass.

The synthesis and evaluation of the polyester resin, and the synthesis and evaluation of the polyurethane resin were carried out in the same manner as in Example 1.

### (Evaluation results)

Evaluation of polyester Reaction time: A Number of colors: < 1 Odor: B Evaluation of polyurethane Reaction time: B Breaking strength of coating film: A Breaking elongation of coating film: A Hydrolysis resistance: A

### (Example 6: Preparation of 1,6-hexanediol composition 6 (1,6-HD composition 6))

A 1,6-hexanediol composition 6 (1,6-HD composition 6) was prepared by adding glycerin to the 1,6-HD composition 3 obtained in Example 3 such that the glycerin content was 1500 ppm by mass.

The synthesis and evaluation of the polyester resin, and the synthesis and evaluation of the polyurethane resin were carried out in the same manner as in Example 1.

### (Evaluation results)

Evaluation of polyester Reaction time: A Number of colors: < 1 Odor: B Evaluation of polyurethane Reaction time: B Breaking strength of coating film: B Breaking elongation of coating film: C Hydrolysis resistance: B

### (Example 7: Preparation of 1,6-hexanediol composition 7 (1,6-HD composition 7))

A 1,6-hexanediol composition 7 (1,6-HD composition 7) was prepared by adding 1,4-cyclohexanediol to the 1,6-HD composition 3 obtained in Example 3 such that the 1,4-cyclohexanediol content was 100 ppm by mass.

The synthesis and evaluation of the polyester resin, and the synthesis and evaluation of the polyurethane resin were carried out in the same manner as in Example 1.

### (Evaluation results)

Evaluation of polyester Reaction time: A Number of colors: < 1 Odor: B Evaluation of polyurethane Reaction time: C Breaking strength of coating film: B Breaking elongation of coating film: B Hydrolysis resistance: B

### (Example 8: Preparation of 1,6-hexanediol composition 8 (1,6-HD composition 8))

A 1,6-hexanediol composition 8 (1,6-HD composition 8) was prepared by adding 6-hydroxyhexanal to the 1,6-HD composition 3 obtained in Example 3 such that the 6-hydroxyhexanal content was 1500 ppm by mass.

The synthesis and evaluation of the polyester resin, and the synthesis and evaluation of the polyurethane resin were carried out in the same manner as in Example 1.

### (Evaluation results)

Evaluation of polyester Reaction time: A Number of colors: 2 Odor: C Evaluation of polyurethane Reaction time: B Breaking strength of coating film: B Breaking elongation of coating film: B Hydrolysis resistance: B

### (Comparative Example 1: Preparation of 1,6-hexanediol composition 9 (1,6-HD composition 9))

A 1,6-hexanediol composition 9 (1,6-HD composition 9) was prepared by adding glucose to the 1,6-HD composition 3 obtained in Example 3 such that the glucose content was 150 ppm by mass.

The synthesis and evaluation of the polyester resin, and the synthesis and evaluation of the polyurethane resin were carried out in the same manner as in Example 1.

### (Evaluation results)

Evaluation of polyester Reaction time: A (bumps occurred) Number of colors: 3 Odor: B

Evaluation of polyurethane Since bumps occurred in the polyester formation, the evaluation was not performed.

### (Comparative Example 2: Preparation of 1,6-hexanediol composition 10 (1,6-HD composition 10))

A 1,6-hexanediol composition 10 (1,6-HD composition 10) was prepared by adding 1,4-cyclohexanediol to the 1,6-HD composition 3 obtained in Example 3 such that the 1,4-cyclohexanediol content was 150 ppm by mass.

The synthesis and evaluation of the polyester resin, and the synthesis and evaluation of the polyurethane resin were carried out in the same manner as in Example 1.

### (Evaluation results)

Evaluation of polyester Reaction time: B Number of colors: < 1 Odor: B Evaluation of polyurethane Reaction time: C Breaking strength of coating film: C Breaking elongation of coating film: C Hydrolysis resistance: D

The results of the Examples and Comparative Examples are summarized in the following Tables 1 to 3.

**[Table 1]**

| | | | Example 1 | Example 2 | Example 3 |
|---|---|---|---|---|---|
| 1,6-HD composition | 1,6-HD composition | | 1 | 2 | 3 |
| | Component (A) | 1,4-Cyclohexanediol | Detection limit or less | Detection limit or less | Detection limit or less |
| | | Glucose | Detection limit or less | Detection limit or less | Detection limit or less |
| | Component (B) | 6-Hydroxyhexanal | 500 ppm | 100 ppm | 10 ppm |
| | Other impurities | Glycerin | 800 ppm | 150 ppm | 5 ppm |
| | | Potassium element | 2 ppm | 1 ppm | Detection limit or less |
| | | Sodium element | 2 ppm | 1 ppm | Detection limit or less |
| | | Total of alkali metal elements | 4 ppm | 2 ppm | Detection limit or less |
| PEs | Composition | Diol | 1,6-HD composition 1 | 1,6-HD composition 2 | 1,6-HD composition 3 |
| | | Dicarboxylic acid | Adipic acid | Adipic acid | Adipic acid |
| | Physical properties | PEs | PEs-A | PEs-B | PEs-C |
| | | Hydroxyl group value (mg KOH/g) | 55.9 | 55.4 | 55.5 |
| | | Acid value (mg KOH/g) | 0.2 | 0.2 | 0.2 |
| | | Number average molecular weight (Mn) | 2000 | 2000 | 2000 |
| | | Reaction time | A | A | A |
| | | Number of colors | 1 | < 1 | < 1 |
| | | Odor | C | B | B |
| PU | Composition | Polyol | PEs-A | PEs-B | PEs-C |
| | | | NPG | NPG | NPG |
| | | Polyisocyanate | TDI | TDI | TDI |
| | Physical properties | PU | PU-A | PU-B | PU-C |
| | | Reaction time | B | B | B |
| | | Breakina strength of coating film | B | B | A |
| | | Breaking elongation of coating film | C | B | A |
| | | Hydrolysis resistance | A | B | B |

**[Table 2]**

| | | | Example 4 | Example 5 | Example 6 |
|---|---|---|---|---|---|
| 1,6-HD composition | 1,6-HD composition | | 4 | 5 | 6 |
| | Component (A) | 1,4-Cyclohexanediol | Detection limit or less | Detection limit or less | Detection limit or less |
| | | Glucose | Detection limit or less | Detection limit or less | Detection limit or less |
| | Component (B) | 6-Hydroxyhexanal | 10 ppm | 10 ppm | 10 ppm |
| | Other impurities | Glycerin | 5 ppm | 150 ppm | 1500 ppm |
| | | Potassium element | 300 ppm | Detection limit or less | Detection limit or less |
| | | Sodium element | 200 ppm | Detection limit or less | Detection limit or less |
| | | Total of alkali metal elements | 500 ppm | Detection limit or less | Detection limit or less |
| PEs | Composition | Diol | 1,6-HD composition 4 | 1,6-HD composition 5 | 1,6-HD composition 6 |
| | | Dicarboxylic acid | Adipic acid | Adipic acid | Adipic acid |
| | Physical properties | PEs | PEs-D | PEs-E | PEs-F |
| | | Hydroxyl group value (mg KOH/g) | 56 | 55.2 | 56.2 |
| | | Acid value (mg KOH/g) | 0.2 | 0.2 | 0.2 |
| | | Number average molecular weight (Mn) | 2000 | 2000 | 1900 |
| | | Reaction time | A | A | A |
| | | Number of colors | < 1 | < 1 | < 1 |
| | | Odor | B | B | B |
| PU | Composition | Polyol | PEs-D | PEs-E | PEs-F |
| | | | NPG | NPG | NPG |
| | | Polyisocyanate | TDI | TDI | TDI |
| | Physical properties | PU | PU-D | PU-E | PU-F |
| | | Reaction time | A | B | B |
| | | Breakina strength of coating film | A | A | B |
| | | Breakina elongation of coating film | A | A | C |
| | | Hydrolysis resistance | A | A | B |

**[Table 3]**

| | | | Example 7 | Example 8 | Comparative Example 1 | Comparative Example 2 |
|---|---|---|---|---|---|---|
| 1,6-HD compositi on | 1,6-HD composition | | 7 | 8 | 9 | 10 |
| | Component (A) | 1,4-Cyclohexanediol | 100 ppm | Detection limit or less | Detection limit or less | 150 ppm |
| | | Glucose | Detection limit or less | Detection limit or less | 150 ppm | Detection limit or less |
| | Component (B) | 6-Hydroxyhexanal | 10 ppm | 1500 ppm | 10 ppm | 10 ppm |
| | Other impurities | Glycerin | 5 ppm | 5 ppm | 5 ppm | 5 ppm |
| | | Potassium element | Detection limit or less | Detection limit or less | Detection limit or less | Detection limit or less |
| | | Sodium element | Detection limit or less | Detection limit or less | Detection limit or less | Detection limit or less |
| | | Total of alkali metal elements | Detection limit or less | Detection limit or less | Detection limit or less | Detection limit or less |
| PEs | Composition | Diol | 1,6-HD composition 7 | 1,6-HD composition 8 | 1,6-HD composition 9 | 1,6-HD composition 10 |
| | | Dicarboxylic acid | Adipic acid | Adipic acid | Adipic acid | Adipic acid |
| | Physical properties | PEs | PEs-G | PEs-H | PEs-I | PEs-J |
| | | Hydroxyl group value (mg KOH/g) | 55.9 | 55.9 | 56.5 | 55.8 |
| | | Acid value (mg KOH/g) | 0.2 | 0.2 | 0.2 | 0.2 |
| | | Number average molecular weight (Mn) | 2000 | 2000 | 1900 | 2000 |
| | | Reaction time | A | A | A (bumps occurred) | B |
| | | Number of colors | < 1 | 2 | 3 | < 1 |
| | | Odor | B | C | B | B |
| PU | Composition | Polyol | PEs-G | PEs-H | PEs-I | PEs-J |
| | | | NPG | NPG | NPG | NPG |
| | | Polyisocyanate | TDI | TDI | TDI | TDI |
| | Physical properties | PU | PU-G | PU-H | PU-I | PU-J |
| | | Reaction time | C | B | Bumps occurred | C |
| | | Breaking strength of coating film | B | B | | C |
| | | Breaking elongation of coating film | B | B | | C |
| | | Hydrolysis resistance | B | B | | D |

From the above-described Examples and Comparative Examples, it was found that the method for producing 1,6-hexanediol composition of the present invention is a method for producing a 1,6-hexanediol composition having a total content of (A) a compound having two or more secondary hydroxyl groups in one molecule of 100 ppm by mass or less, and a total content of (B) 6-hydroxyhexanal and/or a derivative thereof of 1500 ppm by mass, the method including: a step (1) of producing a 1,6-hexanediol composition from 6-hydroxycaproic acid and/or a derivative thereof obtained from a biomass resource-derived raw material; and a step (2) of purifying the 1,6-hexanediol composition obtained in the step (1) by ion exchange and/or distillation, and therefore, a 1,6-hexanediol composition having excellent reactivity can be produced in an environmentally friendly manner.

Furthermore, it was found that a 1,6-hexanediol composition obtained by the method for producing a 1,6-hexanediol composition of the present invention has excellent reactivity, and a polyester obtained using the 1,6-hexanediol composition as a reaction raw material also has excellent reactivity.

Furthermore, it was found that a polyurethane produced using the 1,6-hexanediol composition of the present invention as a reaction raw material has satisfactory tensile strength and tensile elongation.

In the present specification, the number average molecular weight (Mn) of a polyester is a value measured by gel permeation chromatography (GPC) under the following conditions.
Measuring apparatus; HLC-8320GPC manufactured by Tosoh Corporation
Column; TSKgel 4000HXL, TSKgel 3000HXL, TSKgel 2000HXL, TSKgel 1000HXL manufactured by Tosoh Corporation were used by connecting them in series.
Detector; RI (differential refractometer)
Data processing; MULTISTATION GPC-8020 model II manufactured by Tosoh Corporation Measurement conditions; Column temperature 40°C

| |
|---|
| Solvent tetrahydrofuran (THF) |
| Flow rate 0.1 ml/min |

Standard; Monodisperse polystyrene
Sample; A 0.2% tetrahydrofuran solution calculated in terms of resin solid content, filtered through a microfilter (100 µl)
Standard sample: A calibration curve was created using the following standard polystyrenes. (Standard polystyrenes)
"TSKgel standard polystyrene A-500" manufactured by Tosoh Corporation
"TSKgel standard polystyrene A-1000" manufactured by Tosoh Corporation
"TSKgel standard polystyrene A-2500" manufactured by Tosoh Corporation
"TSKgel standard polystyrene A-5000" manufactured by Tosoh Corporation
"TSKgel standard polystyrene F-1" manufactured by Tosoh Corporation
"TSKgel standard polystyrene F-2" manufactured by Tosoh Corporation
"TSKgel standard polystyrene F-4" manufactured by Tosoh Corporation
"TSKgel standard polystyrene F-10" manufactured by Tosoh Corporation
"TSKgel standard polystyrene F-20" manufactured by Tosoh Corporation
"TSKgel standard polystyrene F-40" manufactured by Tosoh Corporation
"TSKgel standard polystyrene F-80" manufactured by Tosoh Corporation
"TSKgel standard polystyrene F-128" manufactured by Tosoh Corporation
"TSKgel standard polystyrene F-288" manufactured by Tosoh Corporation
"TSKgel standard polystyrene F-550" manufactured by Tosoh Corporation

Furthermore, in the present specification, the number average molecular weight (Mn) of a polyurethane is a value measured by gel permeation chromatography (GPC) under the following conditions.
Measuring apparatus: High speed GPC apparatus ("HLC-8220GPC" manufactured by Tosoh Corporation)
Column: The following columns manufactured by Tosoh Corporation were used by connecting them in series.
   "TSKgel G5000" (7.8 mm I.D. × 30 cm) × 1 unit
   "TSKgel G4000" (7.8 mm I.D. × 30 cm) × 1 unit
   "TSKgel G3000" (7.8 mm I.D. × 30 cm) × 1 unit
   "TSKgel G2000" (7.8 mm I.D. × 30 cm) × 1 unit
Detector: RI (differential refractometer)
Column temperature: 40°C
Eluent: Tetrahydrofuran (THF)
Flow rate: 1.0 ml/min
Injection volume: 100 µL (tetrahydrofuran solution having a sample concentration of 0.4% by mass)
Standard sample: The following standard polystyrenes were used to create a calibration curve.

### (Standard polystyrenes)

"TSKgel standard polystyrene A-500" manufactured by Tosoh Corporation
"TSKgel standard polystyrene A-1000" manufactured by Tosoh Corporation
"TSKgel standard polystyrene A-2500" manufactured by Tosoh Corporation
"TSKgel standard polystyrene A-5000" manufactured by Tosoh Corporation
"TSKgel standard polystyrene F-1" manufactured by Tosoh Corporation
"TSKgel standard polystyrene F-2" manufactured by Tosoh Corporation
"TSKgel standard polystyrene F-4" manufactured by Tosoh Corporation
"TSKgel standard polystyrene F-10" manufactured by Tosoh Corporation
"TSKgel standard polystyrene F-20" manufactured by Tosoh Corporation
"TSKgel standard polystyrene F-40" manufactured by Tosoh Corporation
"TSKgel standard polystyrene F-80" manufactured by Tosoh Corporation
"TSKgel standard polystyrene F-128" manufactured by Tosoh Corporation
"TSKgel standard polystyrene F-288" manufactured by Tosoh Corporation
"TSKgel standard polystyrene F-550" manufactured by Tosoh Corporation

## Claims

1. A method for producing a 1,6-hexanediol composition, the method comprising:
a step (1) of producing a 1,6-hexanediol composition from 6-hydroxycaproic acid and/or a derivative thereof obtained from a biomass resource-derived raw material; and
a step (2) of purifying the 1,6-hexanediol composition obtained in the step (1) by ion exchange and/or distillation,
wherein the 1,6-hexanediol composition contains either or both of 1,6-hexanediol and a 1,6-hexanediol derivative, (A) a compound having two or more secondary hydroxyl groups in one molecule, and (B) 6-hydroxyhexanal and/or a derivative thereof,
a content of (A) the compound having two or more secondary hydroxyl groups in one molecule with respect to a total amount of the 1,6-hexanediol composition is 100 ppm by mass or less, and
a total content of (B) 6-hydroxyhexanal and/or a derivative thereof with respect to the total amount of the 1,6-hexanediol composition is 1500 ppm by mass or less.

2. The method for producing a 1,6-hexanediol composition according to claim 1, wherein the step (1) is a step of producing the 1,6-hexanediol composition under conditions at 70°C or lower.

3. The method for producing a 1,6-hexanediol composition according to claim 1, wherein the step (1) is a step of producing the 1,6-hexanediol composition under conditions at 50°C or lower.

4. The method for producing a 1,6-hexanediol composition according to claim 1, wherein the step (1) is a step of producing the 1,6-hexanediol composition using a microorganism.

5. A 1,6-hexanediol composition obtained by the method for producing a 1,6-hexanediol composition according to any one of claims 1 to 4, the 1,6-hexanediol composition comprising:
either or both of 1,6-hexanediol and a 1,6-hexanediol derivative;
(A) a compound having two or more secondary hydroxyl groups in one molecule; and
(B) 6-hydroxyhexanal and/or a derivative thereof,
wherein a total content of (A) the compound having two or more secondary hydroxyl groups in one molecule with respect to a total amount of the 1,6-hexanediol composition is 100 ppm by mass or less, and
a total content of (B) 6-hydroxyhexanal and/or a derivative thereof with respect to the total amount of the 1,6-hexanediol composition is 1500 ppm by mass or less.

6. A polymer produced using the 1,6-hexanediol composition according to claim 5 as a reaction raw material.

7. The polymer according to claim 6, wherein the polymer is a polyester or a polyurethane.
